(19) Europäisches Patentamt
European Patent Office
Office européen des brevets

(11) Publication number: 0 171 977 A1

# (12) EUROPEAN PATENT APPLICATION

(21) Application number: 85305471.6

(22) Date of filing: 31.07.85

(51) Int. Cl.4: C 07 D 209/48
C 07 D 401/12, C 07 D 403/12
C 07 D 413/12, C 07 D 417/12
A 61 K 31/395

(30) Priority: 31.07.84 JP 160399/84

(43) Date of publication of application: 19.02.86 Bulletin 86/8

(84) Designated Contracting States: AT BE CH DE FR GB IT LI NL SE

(71) Applicant: SANKYO COMPANY LIMITED
No. 1-6, 3-chome Nihonbashi Honcho Chuo-ku
Tokyo(JP)

(72) Inventor: Morisawa, Yasuhiro Chemical Research Lab.
Sankyo Co., Ltd. 2-58, 1-chome, Hiromachi
Shinagawa-ku Tokyo(JP)

(72) Inventor: Kataoka, Mitsuru c/o Chemical Research Lab.
Sankyo Co., Ltd. 2-58, 1-chome, Hiromachi
Shinagawa-ku Tokyo(JP)

(72) Inventor: Kumakura, Seiji Biological Research Lab.
Sankyo Co., Ltd. 2-58, 1-chome, Hiromachi
Shinagawa-ku Tokyo(JP)

(72) Inventor: Koike, Hiroyuki Biological Chemical Research Lab.
Sankyo Co., Ltd. 2-58, 1-chome, Hiromachi
Shinagawa-ku Tokyo(JP)

(74) Representative: Gibson, Christian John Robert et al,
Marks & Clerk 57/60 Lincoln's Inn Fields
London WC2A 3LS(GB)

## (54) Phthalimide derivatives, compositions containing them and a process for preparing them.

(57) Compounds of formula (I):

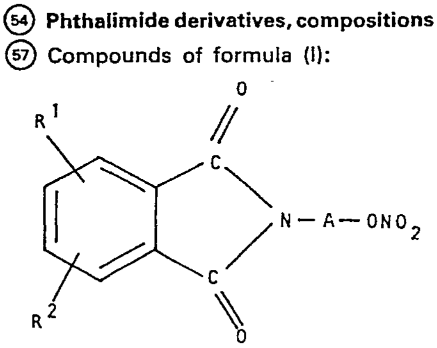

(I)

(wherein $R^2$ and A are a variety of organic groups, optionally hydroxy- or nitrooxy-substituted, and $R^1$ is hydrogen or nitro) are new compounds valuable in the treatment of ischaemic heart disease, especially angina pectoris. They may be prepared by nitrating the corresponding hydroxy compound.

EP 0 171 977 A1

M&C FOLIO: 50522 WANGDOC: 0370H

# PHTHALIMIDE DERIVATIVES, COMPOSITIONS CONTAINING THEM AND A PROCESS FOR PREPARING THEM

The present invention relates to a series of new nitrooxy-substituted phthalimide derivatives which are useful in the treatment of ischaemic cardiac diseases, particularly angina pectoris; the invention also provides pharmaceutical compositions containing these compounds for the treatment of angina pectoris, and a process for preparing them.

Although certain phthalimide derivatives have been described for a variety of medical uses (albeit not the therapy of angina pectoris), none has so far been described having a nitrooxy substituent, as do the compounds of the present invention.

Glyceryl trinitrate is widely used for the treatment of angina pectoris by sublingual administration; however, oral administration of glyceryl trinitrate is of little practical use, since, although glyceryl trinitrate is readily absorbed from the gastrointestinal tract, it is extensively metabolized in the liver, which reduces its bioavailability to the extent that safe doses are of little practical value.

Accordingly, glyceryl trinitrate cannot be used for the prophylaxis of anginal attacks, but can only be used to provide speedy relief from angina pectoris once its symptoms have developed. Moreover, sublingual administration of glyceryl trinitrate can, depending upon the dosage, cause an undesirably high frequency of adverse effects, including hypotension, orthostatic hypotension, reflex tachycardia, throbbing headache, dizziness, nausea and vomiting.

Apart from glyceryl trinitrate, a few other nitrooxy group-containing compounds have been proposed for use in the treatment of angina pectoris, including the recently proposed compound nicorandil, but, despite its disadvantages, glyceryl trinitrate is still regarded as the drug of choice in most cases for the immediate relief of the symptoms of angina pectoris.

It is believed that the effectiveness of glyceryl trinitrate arises from its selective dilative effect on the collateral coronary arteries. Clearly, there is a significant need for a compound which would have a similar type of activity without the disadvantages of glyceryl trinitrate and, in particular, without affecting the heart beat.

We have now discovered a series of compounds which have a selective dilative effect on the collateral coronary arteries, whilst hardly affecting heart beat, which can be administered orally and which have a long duration of activity.

The compounds of the present invention are compounds of formula (I):

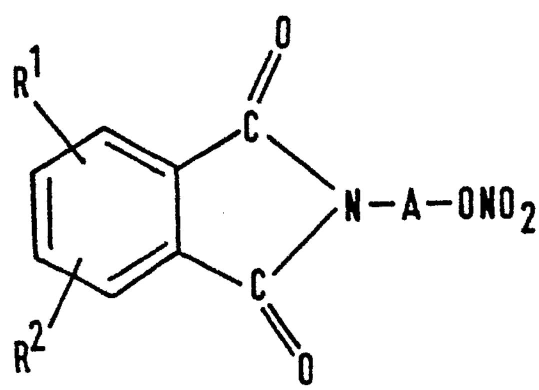

(I)

wherein:

A represents a $C_1$-$C_6$ alkylene group, or a $C_1$-$C_6$ alkylene group having 1 or 2 hydroxy and/or nitrooxy substituents;

$R^1$ represents a hydrogen atom or a nitro group;

$R^2$ represents a hydrogen atom, a $C_1$-$C_6$ alkoxy

group, a $C_1$-$C_6$ alkoxy group having 1 or 2 hydroxy and/or nitrooxy substituents, a hydroxy group, a $C_1$-$C_6$ alkanoylamino group, a halogen atom or a group of formula

$$R^3R^4N\text{-}Y\text{-}B\text{-}O\text{-}$$

or

$$R^3R^4N\text{-}CH_2\text{-}CHR^5\text{-}CH_2\text{-}O\text{-};$$

$R^3$ represents a hydrogen atom or a $C_1$-$C_6$ alkyl group;

$R^4$ represents a hydrogen atom, a $C_1$-$C_6$ alkyl group, a $C_3$-$C_{10}$ cycloalkyl group, a $C_1$-$C_6$ alkanoyl group, a $C_6$-$C_{14}$ carbocyclic aryl group, a substituted $C_6$-$C_{14}$ carbocyclic aryl group, an aralkyl group in which the aryl part is $C_6$-$C_{14}$ carbocyclic aryl and the alkyl part is $C_1$-$C_6$ alkyl or a substituted aralkyl group in which the aryl part is $C_6$-$C_{14}$ carbocyclic aryl and the alkyl part is $C_1$-$C_6$ alkyl;

or $R^3$ and $R^4$, together with the nitrogen atom to which they are attached, represent a heterocyclic group having from 5 to 7 ring atoms of which 1 or 2, including

the nitrogen atom to which $R^3$ and $R^4$ are attached, are nitrogen, oxygen or sulphur hetero-atoms, or such a heterocyclic group which is substituted;

$R^5$ represents a hydroxy or nitrooxy group;

B represents a $C_1$-$C_6$ alkylene group;

Y represents a carbonyl group or a direct bond between B and $-NR^3R^4$; and

the substituents on said substituted aryl, substituted aralkyl and substituted heterocyclic groups are from 1 to 3 substituents selected from $C_1$-$C_4$ alkyl groups, $C_1$-$C_4$ alkoxy groups, halogen atoms, nitro groups, carbamoyl groups, mono- and di-alkylcarbamoyl groups where the or each alkyl part is $C_1$-$C_4$ alkyl, carboxy groups, $C_2$-$C_7$ alkoxycarbonyl groups and, only for substituted heterocyclic groups, oxygen atoms, $C_6$-$C_{14}$ carbocyclic aryl groups, and substituted $C_6$-$C_{14}$ carbocyclic aryl groups;

and pharmaceutically acceptable salts thereof.

The invention also provides a pharmaceutical composition comprising an active ingredient in admixture

with a pharmaceutically acceptable carrier or diluent, wherein the active ingredient is at least one consisting of compound of formula (I) or a pharmaceutically acceptable salt thereof.

The invention still further provides the use of a compound of formula (I) or a pharmaceutically acceptable salt thereof for the manufacture of a medicament for the treatment and prophylaxis of ischaemic heart disease, especially angina pectoris.

The invention also provides a process for preparing the compounds of the invention, as defined more fully hereafter.

In the compounds of the invention, A represents an alkylene group, which may be a straight or branched chain alkylene group, having from 1 to 6 carbon atoms and optionally having 1 or 2 hydroxy and/or nitrooxy substituents. An alkylene group is a group derived by removing two hydrogen atoms from an alkane and these two hydrogen atoms may either be removed from different carbon atoms or from the same carbon atom (alkylene groups in which the two hydrogen atoms are removed from the same carbon atom are sometimes referred to as "alkylidene" groups). Examples of unsubstituted alkylene groups include the methylene, ethylene,

trimethylene, propylene (which may be the 1-methylethylene or 2-methylethylene group), tetramethylene, 1,2-butylene (which may be the 1-ethylethylene or 2-ethylethylene group), pentamethylene and hexamethylene groups. Such groups may be unsubstituted or may have a single hydroxy or nitrooxy substituent, two hydroxy substituents, two nitrooxy substituents or one hydroxy and one nitrooxy substituent. We particularly prefer compounds which have a single hydroxy or single nitrooxy substituent, more preferably a single nitrooxy substituent, on any one of the aforementioned alkylene groups. Examples of such substituted alkylene groups include the 2-hydroxytrimethylene and 2-nitrooxytrimethylene groups.

$R^1$ represents a hydrogen atom or a nitro group. Where $R^1$ represents a nitro group, it may be at the 3- or 4-position or (where $R^2$ represents a group other than the hydrogen atom) at the 5- or 6-position. Where $R^2$ is a group other than the hydrogen atom and is, as is preferred, at the 4-position and where $R^1$ represents a nitro group, this nitro group represented by $R^1$ is preferably at the 3- or 5-position.

$R^2$ can represent a hydrogen atom, a $C_1$-$C_6$ alkoxy group (which may have 1 or 2 hydroxy and/or nitrooxy substituents), a hydroxy group, a $C_1$-$C_6$

alkanoylamino group, a halogen atom or a group of formula

$R^3R^4N-Y-B-O-$ or $R^3R^4N-CH_2-CH(R^5)-CH_2-O-$.

Where $R^2$ represents a $C_1-C_6$ alkoxy group, this may be a straight or branched chain alkoxy group and is more preferably an alkoxy group having from 1 to 4 carbon atoms, which alkoxy group may be substituted or unsubstituted. Examples of such unsubstituted alkoxy groups include the methoxy, ethoxy, propoxy, isopropoxy, butoxy, isobutoxy, sec-butoxy, t-butoxy, pentyloxy, isopentyloxy, neopentyloxy, sec-pentyloxy, t-pentyloxy or hexyloxy groups, of which the methoxy, ethoxy, propoxy, isopropoxy, butoxy and isobutoxy groups are preferred. Where the alkoxy group, e.g. any one of those exemplified above, is substituted, it may have a single hydroxy substituent, a single nitrooxy substituent, two hydroxy substituents, two nitrooxy substituents or one hydroxy substituent and one nitrooxy substituent. Preferred examples of such substituted alkoxy groups are the 2-hydroxy-3-nitrooxypropoxy and 2,3-dinitrooxypropoxy groups.

Where $R^2$ represents an alkanoylamino group, this has from 1 to 6, preferably from 2 to 4, carbon atoms and the alkanoyl group may be a straight or branched chain group. Examples of such alkanoylamino groups

include the formamido, acetamido, propionamido, butyramido, isobutyramido, valeramido, isovaleramido, pivalamido or hexanamido groups, of which the acetamido, propionamido, butyramido and isobutyramido groups are preferred.

Where $R^2$ represents a halogen atom, this is preferably a fluorine, chlorine or bromine atom.

Where $R^2$ represents a group of formula $R^3R^4N-Y-B-O-$ or $R^3R^4N-CH_2-CH(R^5)-CH_2-O-$, $R^3$ can represent a hydrogen atom or a $C_1-C_6$ alkyl group and $R^4$ can represent a hydrogen atom, a $C_1-C_6$ alkyl group, a $C_3-C_{10}$ cycloalkyl group, a $C_1-C_6$ alkanoyl group, a $C_6-C_{14}$ carbocyclic aryl group (which may be substituted or unsubstituted) or an aralkyl group (which may be substituted or unsubstituted).

Where $R^3$ or $R^4$ represents an alkyl group, this is a straight or branched chain alkyl group having from 1 to 6, preferably from 1 to 4, carbon atoms and, where $R^3$ and $R^4$ both represent such an alkyl group, the alkyl groups represented by $R^3$ and $R^4$ may be the same or different, but are, for convenience, preferably the same. Examples of such alkyl groups include the methyl, ethyl, propyl, isopropyl, butyl, isobutyl,

sec-butyl, t-butyl, pentyl, isopentyl, sec-pentyl, neopentyl, t-pentyl or hexyl groups, of which the methyl, ethyl, propyl, isopropyl, butyl and isobutyl groups are preferred. In the case of the bulkier alkyl groups, steric hindrance may prevent two such groups being attached to the same nitrogen atom, in which case, we then prefer that one of $R^3$ and $R^4$ should represent a hydrogen atom.

Where $R^4$ represents a cycloalkyl group, this may be monocyclic or polycyclic and may have from 3 to 10, preferably from 3 to 6, ring atoms. Preferred such cycloalkyl groups include the cyclopropyl, cyclobutyl, cyclopentyl and cyclohexyl groups.

Where $R^4$ represents an alkanoyl group, this has from 1 to 6, preferably from 2 to 4, carbon atoms and may be a straight or branched chain alkanoyl group. Examples of such alkanoyl groups include the formyl, acetyl, propionyl, butyryl, isobutyryl, valeryl, isovaleryl, pivaloyl and hexanoyl groups, of which the acetyl, propionyl, butyryl and isobutyryl groups are preferred.

Where $R^4$ represents an aryl group, this is a carbocyclic aryl group having from 6 to 14 ring carbon atoms and may be substituted or unsubstituted. Examples

of unsubstituted aryl groups include the phenyl, 1-naphthyl and 2-naphthyl groups. Where the aryl group is substituted, the substituents may be from 1 to 3 substituents selected from those defined above, but are preferably $C_1$-$C_4$ alkyl groups (examples of which have been given in relation to $R^3$ and $R^4$), or $C_1$-$C_4$ alkoxy groups or halogen atoms (examples of which have been given above in relation to the similar groups and atoms which may be represented by $R^2$) or nitro groups. Preferred such substituted aryl groups include the o-tolyl, m-tolyl, p-tolyl, o-methoxyphenyl, m-methoxyphenyl, p-methoxyphenyl, o-nitrophenyl, m-nitrophenyl, p-nitrophenyl, o-chlorophenyl, m-chlorophenyl and p-chlorophenyl groups.

Where $R^4$ represents an aralkyl group, the aryl part is a $C_6$-$C_{14}$, preferably $C_6$-$C_{10}$, carbocyclic aryl group, which may be substituted or unsubstituted, and the alkyl part is a $C_1$-$C_6$, preferably $C_1$-$C_3$, alkyl group. Preferred unsubstituted aralkyl groups are the benzyl, phenethyl and 3-phenylpropyl groups. Where the aralkyl group is substituted, it may have from 1 to 3 substituents selected from those defined above, but the preferred substituents are $C_1$-$C_4$ alkyl groups (examples of which have been given in relation to $R^3$ and $R^4$) and $C_1$-$C_4$ alkoxy groups and halogen atoms (examples of which have been given in relation to the

similar groups and atoms which may be represented by $R^2$). Preferred such substituted aralkyl groups are the benzyl, o-methylbenzyl, m-methylbenzyl, p-methylbenzyl, o-methoxybenzyl, m-methoxybenzyl, p-methoxybenzyl, o-chlorobenzyl, m-chlorobenzyl and p-chlorobenzyl groups.

Alternatively, $R^3$ and $R^4$, together with the nitrogen atom to which they are attached, i.e. the group $R^3R^4N-$, may represent a heterocyclic group having from 5 to 7 ring atoms of which 1 or 2 (including the nitrogen atom shown) are nitrogen, oxygen or sulphur hetero-atoms. More preferably, the heterocyclic group has 5 or 6 ring atoms of which 1 or 2 (including the nitrogen atom shown) are said hetero-atoms. The heterocyclic group may be substituted or unsubstituted. Examples of preferred unsubstituted heterocyclic groups include the 1-pyrrolidinyl, piperidino, morpholino, 1-piperazinyl, 3-thiazolidinyl and perhydro-1,4-thiazin-4-yl groups.

Where such a heterocyclic group is substituted, it may have from 1 to 3 substituents selected from those defined above. However, preferred substituents are: $C_1$-$C_4$ alkyl groups, such as those exemplified above in relation to $R^3$ and $R^4$; aryl groups which may be substituted or unsubstituted, such as those exemplified

above in relation to $R^4$; $C_2$-$C_7$, preferably $C_2$-$C_4$, alkoxycarbonyl groups in which the alkoxy part may be any one of those alkoxy groups exemplified above in relation to $R^2$, preferably a methoxycarbonyl or ethoxycarbonyl group; and oxygen atoms. Where an oxygen atom is a substituent on a ring carbon atom, it constitutes an oxo group (i.e. a doubly bonded oxygen atom). Where one or two oxygen atoms are bonded to a ring sulphur atom, this is a coordinate bond, to form with the sulphur atom a sulphoxide group (>SO, one oxygen atom) or a sulphone group (>$SO_2$, two oxygen atoms). Preferred examples of such substituted heterocyclic groups include the 2-oxo-1-pyrrolidinyl, 2-oxopiperidino, 4-oxopiperidino, 1-oxoperhydro-1,4-thiazin-4-yl, 1,1-dioxoperhydro-1,4-thiazin-4-yl, 2-methylpiperidino, 4-methylpiperidino, 2,6-dimethylpiperidino, 2-ethylpiperidino, 4-methyl-1-piperazinyl, 4-phenyl-1-piperazinyl, 4-*p*-methylphenyl-1-piperazinyl, 4-*p*-methoxyphenyl-1-piperazinyl, 4-*p*-chlorophenyl-1-piperazinyl, 2-methoxycarbonyl-1-pyrrolidinyl, 2-ethoxycarbonyl-1-pyrrolidinyl and 4-ethoxycarbonyl-1-piperazinyl groups.

Where $R^2$ represents a group of formula $R^3R^4N$-Y-B-O-, B represents a straight or branched chain alkylene group having from 1 to 6 carbon atoms, for example a methylene, ethylene, trimethylene,

propylene (which may be 1-methylethylene or 2-methylethylene), tetramethylene, 1,2-butylene (which may be 1-ethylethylene or 2-ethylethylene), pentamethylene or hexamethylene group.

Y represents a direct bond between the group represented by $R^3R^4N-$ and the alkylene group represented by B or a carbonyl (>C=O) group, preferably such a direct bond.

Where $R^2$ represents a group of formula $R^3R^4N-CH_2-CH(R^5)-CH_2-O-$, $R^3$, $R^4$ and the group $R^3R^4N-$ are as defined above and may be any one of those groups and atoms exemplified above. The group $R^5$ may be a hydroxy group or a nitrooxy group.

Where $R^2$ represents any one of the groups defined above (other than the hydrogen atom), it may be at the 3- or 4-position or (where $R^1$ represents a nitro group) at the 5- or 6-position. We particularly prefer that $R^1$ should represent one of the groups defined above at the 3- or 4-position, most preferably the 4-position.

Particularly preferred compounds of the present invention are those compounds in which:

A represents an unsubstituted ethylene, propylene or trimethylene group;

$R^1$ represents a hydrogen atom or a nitro group;

$R^2$ represents, at the 3- or 4-position, a $C_2$-$C_4$ alkoxy group, a $C_2$-$C_4$ alkoxy group having 1 or 2 hydroxy and/or nitrooxy substituents, a hydroxy group, a $C_2$-$C_4$ alkanoylamino group, a halogen atom, a group of formula $R^3R^4N$-Y-B-O- or a group of formula $R^3R^4N-CH_2-CH(R^5)-CH_2-O-$;

$R^3$ represents a hydrogen atom or a $C_1$-$C_4$ alkyl group;

$R^4$ represents a hydrogen atom, a $C_1$-$C_4$ alkyl group, a $C_5$ or $C_6$ cycloalkyl group, a $C_2$-$C_4$ alkanoyl group, a phenyl group, a substituted phenyl group, or an aralkyl group in which the aryl part is a phenyl group or a substituted phenyl group and the alkyl part is a $C_1$-$C_3$ alkyl group;

or $R^3$ and $R^4$, together with the nitrogen atom to which they are attached, represent a heterocyclic group having 5 or 6 ring atoms of which 1 or 2, including the nitrogen atom to which $R^3$ and $R^4$ are attached, are nitrogen, oxygen or sulphur hetero-atoms, or such a

heterocyclic group which is substituted;

$R^5$ represents a hydroxy or nitrooxy group;

B represents an ethylene, propylene or trimethylene group;

Y represents a direct bond between the alkylene group represented by B and the group $-NR^3R^4$; and

the substituents are as defined above;

and pharmaceutically acceptable salts thereof.

The most preferred compounds of the present invention are those in which:

A represents an ethylene group;

$R^1$ is as defined above;

$R^2$ represents said group of formula $R^3R^4N-Y-B-O-$ at the 3- or 4-position, preferably the 4-position;

$R^3$ and $R^4$ both represent methyl groups or $R^3$ and $R^4$ both represent ethyl groups;

Y represents said direct bond; and

B represents an ethylene or trimethylene group;

and pharmaceutically acceptable salts thereof.

When the compounds of formula (I) are basic [i.e. when $R^2$ represents a group of formula $R^3R^4N-Y-B-O-$ or $R^3R^4N-CH_2-CH(R^5)-CH_2-O-$], they can form acid addition salts. The acids employed to form such salts may be chosen from a virtually unlimited range, the only restriction being that, where the compounds are to be employed for pharmaceutical use, the resulting salts should be pharmaceutically acceptable which, as is well-known, means that the salts should not have reduced, or unacceptably reduced, activity or increased, or unacceptably increased, toxicity as compared with the free acids. Where, however, the compounds are not intended for pharmaceutical use (e.g. if they are intended for use as intermediates), even this restriction does not apply. Suitable pharmaceutically acceptable acid addition salts include salts with: mineral acids, such as hydrochloric acid, hydrobromic acid, sulphuric acid, nitric acid, phosphoric acid or metaphosphoric acid; and salts with organic carboxylic acids, such as acetic acid, oxalic acid, tartaric acid, citric acid, succinic acid, fumaric acid, maleic acid,

gluconic acid or benzoic acid.

It will be appreciated that certain of the substituents which may be represented by A and $R^2$ can contain asymmetric carbon atoms and, accordingly, the compounds of the invention may contain one or more such asymmetric carbon atoms and, as a result, various optical isomers and diastereoisomers may be possible. Although all of the optical isomers and mixtures of such isomers of the compounds of the invention are indicated herein by a single formula, the present invention envisages both the individual isolated isomers and mixtures (which may be racemates) of these isomers. Where the compounds of the invention are prepared in the form of mixtures of isomers, these may, if desired, be separated by conventional resolution techniques; alternatively, they may be employed as such, as mixtures.

Typical examples of compounds of the present invention are given in the following list; hereafter, where appropriate, the compounds of the invention are identified by the numbers assigned to them in this list.

1. N-(2-nitrooxyethyl)-3-(2-N,N-dimethylaminoethoxy)-phthalimide

2. N-(2-nitrooxyethyl)-3-(2-N,N-diethylaminoethoxy)-phthalimide

3. **N**-(2-nitrooxyethyl)-3-(3-**N**,**N**-dimethylaminopropoxy)-phthalimide

4. **N**-(2-nitrooxyethyl)-3-(3-**N**,**N**-diethylaminopropoxy)-phthalimide

5. **N**-(2-nitrooxyethyl)-3-[2-(1-pyrrolidinyl)ethoxy]-phthalimide

6. **N**-(2-nitrooxyethyl)-3-[3-(1-pyrrolidinyl)propoxy]-phthalimide

7. **N**-(2-nitrooxyethyl)-3-(2-piperidinoethoxy)phthalimide

8. **N**-(2-nitrooxyethyl)-3-(1-pyrrolidinylcarbonyl-methoxy)phthalimide

9. **N**-(2-nitrooxyethyl)-3-[2-(1-pyrrolidinylcarbonyl)-ethoxy]phthalimide

10. **N**-(2-nitrooxyethyl)-3-(2-**N**-benzyl-**N**-methylamino-ethoxy)phthalimide

11. **N**-(2-nitrooxyethyl)-3-[2-(4-ethoxycarbonyl-1-piperazinyl)ethoxy]phthalimide

12. N-(2-nitrooxyethyl)-3-[2-(4-methyl-1-piperazinyl)-ethoxy]phthalimide

13. N-(3-nitrooxypropyl)-3-(2-N,N-dimethylaminoethoxy)-phthalimide

14. N-(3-nitrooxypropyl)-3-(2-N,N-diethylaminoethoxy)-phthalimide

15. N-(3-nitrooxypropyl)-3-(2-N,N-dimethylamino-propoxy)phthalimide

16. N-(3-nitrooxypropyl)-3-(2-N,N-diethylaminopropoxy)-phthalimide

17. N-(3-nitrooxypropyl)-3-[2-(1-pyrrolidinyl)ethoxy]-phthalimide

18. N-(3-nitrooxypropyl)-3-(2-piperidinoethoxy)-phthalimide

19. N-(3-nitrooxypropyl)-3-(2-morpholinoethoxy)-phthalimide

20. N-(3-nitrooxypropyl)-3-[3-(1-pyrrolidinyl)-propoxy]phthalimide

21. N-(4-nitrooxybutyl)-3-(2-N,N-diethylaminoethoxy)-phthalimide

22. N-(4-nitrooxybutyl)-3-[2-(1-pyrrolidinyl)ethoxy]-phthalimide

23. N-(4-nitrooxybutyl)-3-(3-N,N-diethylaminopropoxy)-phthalimide

24. N-(2-nitrooxyethyl)-4-(2-N,N-dimethylaminoethoxy)-phthalimide

25. N-(2-nitrooxyethyl)-4-(2-N,N-diethylaminoethoxy)-phthalimide

26. N-(2-nitrooxyethyl)-4-(2-N,N-dipropylaminoethoxy)-phthalimide

27. N-(2-nitrooxyethyl)-4-(2-N,N-diisopropylamino-ethoxy)phthalimide

28. N-(2-nitrooxyethyl)-4-(2-N-butyl-N-methylamino-ethoxy)phthalimide

29. N-(2-nitrooxyethyl)-4-(2-N-butyl-N-ethylamino-ethoxy)phthalimide

30. N-(2-nitrooxyethyl)-4-(2-N-benzyl-N-methylaminoethoxy)phthalimide

31. N-(2-nitrooxyethyl)-4-(2-N-benzyl-N-ethylaminoethoxy)phthalimide

32. N-(2-nitrooxyethyl)-4-(2-N-benzyl-N-propylaminoethoxy)phthalimide

33. N-(2-nitrooxyethyl)-4-(2-N-benzyl-N-isopropylaminoethoxy)phthalimide

34. N-(2-nitrooxyethyl)-4-(2-N-ethyl-N-p-methoxybenzylaminoethoxy)phthalimide

35. N-(2-nitrooxyethyl)-4-(2-N-ethyl-N-p-methylbenzylaminoethoxy)phthalimide

36. N-(2-nitrooxyethyl)-4-(2-N-p-chlorobenzyl-N-ethylaminoethoxy)phthalimide

37. N-(2-nitrooxyethyl)-4-(2-N-methylanilinoethoxy)phthalimide

38. N-(2-nitrooxyethyl)-4-(2-N-ethylanilinoethoxy)phthalimide

39. N-(2-nitrooxyethyl)-4-(2-N-methyl-p-anisidinoethoxy)phthalimide

40. N-(2-nitrooxyethyl)-4-(2-N-ethyl-m-toluidinoethoxy)phthalimide

41. N-(2-nitrooxyethyl)-4-(2-N-ethyl-p-chloroanilinoethoxy)phthalimide

42. N-(2-nitrooxyethyl)-4-(2-N-cyclohexyl-N-methylaminoethoxy)phthalimide

43. N-(2-nitrooxyethyl)-4-(2-N-cyclohexyl-N-isopropylaminoethoxy)phthalimide

44. N-(2-nitrooxyethyl)-4-(2-N-cyclopropyl-N-ethylaminoethoxy)phthalimide

45. N-(2-nitrooxyethyl)-4-(2-N-acetyl-N-methylaminoethoxy)phthalimide

46. N-(2-nitrooxyethyl)-4-(2-N-acetyl-N-ethylaminoethoxy)phthalimide

47. N-(2-nitrooxyethyl)-4-(2-N-methyl-N-propionylaminoethoxy)phthalimide

48. N-(2-nitrooxyethyl)-4-(2-N-ethyl-N-propionylamino-ethoxy)phthalimide

49. N-(2-nitrooxyethyl)-4-(1-pyrrolidinylcarbonyl-methoxy)phthalimide

50. N-(2-nitrooxyethyl)-4-[2-(1-pyrrolidinylcarbonyl)-ethoxy]phthalimide

51. N-(2-nitrooxyethyl)-4-(3-N,N-dimethylamino-propoxy)phthalimide

52. N-(2-nitrooxyethyl)-4-(3-N,N-diethylaminopropoxy)-phthalimide

53. N-(2-nitrooxyethyl)-4-(2-N,N-dimethylaminopropoxy)-phthalimide

54. N-(2-nitrooxyethyl)-4-(3-N,N-dimethylamino-2-methylpropoxy)phthalimide

55. N-(2-nitrooxyethyl)-4-[2-(1-pyrrolidinyl)ethoxy]-phthalimide

56. N-(2-nitrooxyethyl)-4-[2-(2-oxo-1-pyrrolidinyl)-ethoxy]phthalimide

57. N-(2-nitrooxyethyl)-4-[2-(2-methoxycarbonyl-1-pyrrolidinyl)ethoxy]phthalimide

58. N-(2-nitrooxyethyl)-4-[3-(1-pyrrolidinyl)propoxy]-phthalimide

59. N-(2-nitrooxyethyl)-4-(2-piperidinoethoxy)-phthalimide

60. N-(2-nitrooxyethyl)-4-[2-(2-oxopiperidino)ethoxy]-phthalimide

61. N-(2-nitrooxyethyl)-4-[2-(4-oxopiperidino)ethoxy]-phthalimide

62. N-(2-nitrooxyethyl)-4-[2-(2-methylpiperidino)-ethoxy]phthalimide

63. N-(2-nitrooxyethyl)-4-[2-(4-methylpiperidino)-ethoxy]phthalimide

64. N-(2-nitrooxyethyl)-4-(3-piperidinopropoxy)-phthalimide

65. N-(2-nitrooxyethyl)-4-(2-morpholinoethoxy)-phthalimide

66. N-(2-nitrooxyethyl)-4-(3-morpholinopropoxy)-phthalimide

67. N-(2-nitrooxyethyl)-4-[2-(4-ethoxycarbonyl-1-piperazinyl)ethoxy]phthalimide

68. N-(2-nitrooxyethyl)-4-[2-(4-methyl-1-piperazinyl)-ethoxy]phthalimide

69. N-(2-nitrooxyethyl)-4-[2-(4-phenyl-1-piperazinyl)-ethoxy]phthalimide

70. N-(2-nitrooxyethyl)-4-[2-(4-p-tolyl-1-piperazinyl)-ethoxy]phthalimide

71. N-(2-nitrooxyethyl)-4-[2-(4-p-methoxyphenyl-1-piperazinyl)ethoxy]phthalimide

72. N-(2-nitrooxyethyl)-4-[2-(4-o-methoxyphenyl-1-piperazinyl)ethoxy]phthalimide

73. N-(2-nitrooxyethyl)-4-[2-(4-p-chlorophenyl-1-piperazinyl)ethoxy]phthalimide

74. N-(2-nitrooxyethyl)-4-[3-(4-ethoxycarbonyl-1-piperazinyl)propoxy]phthalimide

75. N-(2-nitrooxyethyl)-4-[3-(4-methyl-1-piperazinyl)-propoxy]phthalimide

76. N-(3-nitrooxypropyl)-4-(2-N,N-dimethylaminoethoxy)-phthalimide

77. N-(3-nitrooxypropyl)-4-(2-N,N-diethylaminoethoxy)-phthalimide

78. N-(3-nitrooxypropyl)-4-(2-N,N-dipropylaminoethoxy)-phthalimide

79. N-(3-nitrooxypropyl)-4-(2-N,N-diisopropylamino-ethoxy)phthalimide

80. N-(3-nitrooxypropyl)-4-(2-N-butyl-N-methylamino-ethoxy)phthalimide

81. N-(3-nitrooxypropyl)-4-(2-N-butyl-N-ethylamino-ethoxy)phthalimide

82. N-(3-nitrooxypropyl)-4-(2-N-benzyl-N-methylamino-ethoxy)phthalimide

83. N-(3-nitrooxypropyl)-4-(2-N-benzyl-N-ethylamino-ethoxy)phthalimide

84. N-(3-nitrooxypropyl)-4-(2-N-benzyl-N-propylaminoethoxy)phthalimide

85. N-(3-nitrooxypropyl)-4-(2-N-methylanilinoethoxy)phthalimide

86. N-(3-nitrooxypropyl)-4-(2-N-ethylanilinoethoxy)phthalimide

87. N-(3-nitrooxypropyl)-4-(2-N-cyclohexyl-N-methylaminoethoxy)phthalimide

88. N-(3-nitrooxypropyl)-4-(2-N-acetyl-N-methylaminoethoxy)phthalimide

89. N-(3-nitrooxypropyl)-4-(2-N-acetyl-N-ethylaminoethoxy)phthalimide

90. N-(3-nitrooxypropyl)-4-(3-N,N-dimethylaminopropoxy)phthalimide

91. N-(3-nitrooxypropyl)-4-(3-N,N-diethylaminopropoxy)phthalimide

92. N-(3-nitrooxypropyl)-4-[2-(1-pyrrolidinyl)ethoxy]phthalimide

93. N-(3-nitrooxypropyl)-4-[2-(2-oxo-1-pyrrolidinyl)-ethoxy]phthalimide

94. N-(3-nitrooxypropyl)-4-[3-(1-pyrrolidinyl)propoxy]-phthalimide

95. N-(3-nitrooxypropyl)-4-(2-piperidinoethoxy)-phthalimide

96. N-(3-nitrooxypropyl)-4-[2-(2-oxopiperidino)ethoxy]-phthalimide

97. N-(3-nitrooxypropyl)-4-[2-(2-methylpiperidino)-ethoxy]phthalimide

98. N-(3-nitrooxypropyl)-4-[2-(4-methylpiperidino)-ethoxy]phthalimide

99. N-(3-nitrooxypropyl)-4-(3-piperidinopropoxy)-phthalimide

100. N-(3-nitrooxypropyl)-4-(2-morpholinoethoxy)-phthalimide

101. N-(3-nitrooxypropyl)-4-[2-(4-ethoxycarbonyl-1-piperazinyl)ethoxy]phthalimide

102. N-(3-nitrooxypropyl)-4-[2-(4-methyl-1-piperazinyl)ethoxy]phthalimide

103. N-(3-nitrooxypropyl]-4-[2-(4-phenyl-1-piperazinyl)ethoxy]phthalimide

104. N-(3-nitrooxypropyl)-4-[3-(4-ethoxycarbonyl-1-piperazinyl)propoxy]phthalimide

105. N-(3-nitrooxypropyl)-4-[3-(4-methyl-1-piperazinyl)propoxy]phthalimide

106. N-(4-nitrooxybutyl)-4-(2-N,N-dimethylaminoethoxy)-phthalimide

107. N-(4-nitrooxybutyl)-4-(2-N,N-diethylaminoethoxy)-phthalimide

108. N-(4-nitrooxybutyl)-4-(2-N,N-dipropylaminoethoxy)-phthalimide

109. N-(4-nitrooxybutyl)-4-(2-N,N-diisopropylaminoethoxy)phthalimide

110. N-(4-nitrooxybutyl)-4-(2-N-butyl-N-methylaminoethoxy)phthalimide

111. N-(4-nitrooxybutyl)-4-(2-N-butyl-N-ethylamino-ethoxy)phthalimide

112. N-(4-nitrooxybutyl)-4-(2-N-benzyl-N-methylamino-ethoxy)phthalimide

113. N-(4-nitrooxybutyl)-4-(2-N-benzyl-N-ethylamino-ethoxy)phthalimide

114. N-(4-nitrooxybutyl)-4-[2-(N-methylanilino)ethoxy]-phthalimide

115. N-(4-nitrooxybutyl)-4-(3-N,N-dimethylamino-propoxy)phthalimide

116. N-(4-nitrooxybutyl)-4-[2-(1-pyrrolidinyl)ethoxy]-phthalimide

117. N-(4-nitrooxybutyl)-4-[2-(2-oxo-1-pyrrolidinyl)-ethoxy]phthalimide

118. N-(4-nitrooxybutyl)-4-(2-piperidinoethoxy)-phthalimide

119. N-(4-nitrooxybutyl)-4-[2-(4-methylpiperidino)-ethoxy]phthalimide

120. N-(4-nitrooxybutyl)-4-(3-N,N-diethylaminopropoxy)-phthalimide

121. N-(5-nitrooxypentyl)-4-(2-N,N-diethylaminoethoxy)-phthalimide

122. N-(5-nitrooxypentyl)-4-[2-(1-pyrrolidinyl)ethoxy]-phthalimide

123. N-(5-nitrooxypentyl)-4-(2-piperidinoethoxy)-phthalimide

124. N-(6-nitrooxyhexyl)-4-(2-N,N-diethylaminoethoxy)-phthalimide

125. N-(6-nitrooxyhexyl)-4-[2-(1-pyrrolidinyl)ethoxy]-phthalimide

126. N-(1-methyl-2-nitrooxyethyl)-4-(2-N,N-diethyl-aminoethoxy)phthalimide

127. N-(1-methyl-2-nitrooxyethyl)-4-[2-(1-pyrrolidinyl)ethoxy]phthalimide

128. N-(2-nitrooxypropyl)-4-(2-N,N-dimethylamino-ethoxy)phthalimide

129. N-(2-nitrooxypropyl)-4-(2-N,N-diethylaminoethoxy)-phthalimide

130. N-(2-nitrooxypropyl)-4-[2-(1-pyrrolidinyl)ethoxy]-phthalimide

131. N-(2-nitrooxypropyl)-4-[3-(1-pyrrolidinyl)-propoxy]phthalimide

132. N-(2-nitrooxypropyl)-4-(2-piperidinoethoxy)-phthalimide

133. N-(2-nitrooxypropyl)-4-[2-(1-pyrrolidinyl)ethoxy]-5-nitrophthalimide

134. N-(2,3-dinitrooxypropyl)-4-(2-N,N-diethylamino-ethoxy)phthalimide

135. N-(2,3-dinitrooxypropyl)-4-[2-(1-pyrrolidinyl)-ethoxy]phthalimide

136. N-(2-nitrooxyethyl)-4-(2-N,N-dimethylamino-ethoxy)-5-nitrophthalimide

137. N-(2-nitrooxyethyl)-4-(2-N,N-diethylaminoethoxy)-5-nitrophthalimide

138. N-(2-nitrooxyethyl)-4-(2-N,N-dipropylamino-ethoxy)-5-nitrophthalimide

139. N-(2-nitrooxyethyl)-4-[2-(1-pyrrolidinyl)ethoxy]-5-nitrophthalimide

140. N-(2-nitrooxyethyl)-4-(2-piperidinoethoxy)-5-nitrophthalimide

141. N-(2-nitrooxyethyl)-4-(3-N,N-dimethylamino-propoxy)-3-nitrophthalimide

142. N-(2-nitrooxyethyl)-4-(3-N,N-dimethylamino-propoxy)-5-nitrophthalimide

143. N-(2-nitrooxyethyl)-4-(3-N,N-diethylaminopropoxy)-5-nitrophthalimide

144. N-(2-nitrooxyethyl)-4-[3-N,N-dipropylamino-propoxy)-5-nitrophthalimide

145. N-(2-nitrooxyethyl)-4-[3-(1-pyrrolidinyl)propoxy]-3-nitrophthalimide

146. N-(2-nitrooxyethyl)-4-[3-(1-pyrrolidinyl)propoxy]-5-nitrophthalimide

147. N-(3-nitrooxypropyl)-4-(2-N,N-dimethylamino-ethoxy)-5-nitrophthalimide

148. N-(3-nitrooxypropyl)-4-(2-N,N-diethylaminoethoxy)-5-nitrophthalimide

149. N-(3-nitrooxypropyl)-4-(2-N,N-dipropylamino-ethoxy)-5-nitrophthalimide

150. N-(3-nitrooxypropyl)-4-[2-(1-pyrrolidinyl)ethoxy]-5-nitrophthalimide

151. N-(3-nitrooxypropyl)-4-(2-piperidinoethoxy)-5-nitrophthalimide

152. N-(3-nitrooxypropyl)-4-(3-N,N-dimethylamino-propoxy)-5-nitrophthalimide

153. N-(3-nitrooxypropyl)-4-(3-N,N-diethylamino-propoxy)-5-nitrophthalimide

154. N-(3-nitrooxypropyl)-4-[3-(1-pyrrolidinyl)-propoxy]-5-nitrophthalimide

155. N-(2-nitrooxyethyl)phthalimide

156. N-(3-nitrooxypropyl)phthalimide

157. N-(2-nitrooxyethyl)-3-nitrophthalimide

158. N-(2-nitrooxyethyl)-4-nitrophthalimide

159. N-(3-nitrooxypropyl)-3-nitrophthalimide

160. N-(3-nitrooxypropyl)-4-nitrophthalimide

161. N-(2-nitrooxyethyl)-4-chlorophthalimide

162. N-(3-nitrooxypropyl)-4-chlorophthalimide

163. N-(2-nitrooxyethyl)-3-hydroxyphthalimide

164. N-(2-nitrooxyethyl)-4-hydroxyphthalimide

165. N-(3-nitrooxypropyl)-3-hydroxyphthalimide

166. N-(3-nitrooxypropyl)-4-hydroxyphthalimide

167. N-(2-nitrooxyethyl)-3-acetamidophthalimide

168. N-(2-nitrooxyethyl)-4-acetamidophthalimide

169. N-(3-nitrooxypropyl)-3-acetamidophthalimide

170. N-(3-nitrooxypropyl)-4-acetamidophthalimide

171. N-(2-nitrooxyethyl)-4-[2-(2,6-dimethylpiperidino)-ethoxy]phthalimide

172. N-(3-nitrooxypropyl)-4-[2-(2,6-dimethylpiperidino)ethoxy]phthalimide

173. N-(2-nitrooxyethyl)-4-(2-acetamidoethoxy)-phthalimide

174. N-(2-nitrooxyethyl)-4-(2-propionamidoethoxy)-phthalimide

175. N-(2-nitrooxyethyl)-4-(2-butyramidoethoxy)-phthalimide

176. N-(3-nitrooxypropyl)-4-(2-acetamidoethoxy)-phthalimide

177. N-(3-nitrooxypropyl)-4-(2-propionamidoethoxy)-phthalimide

178. N-(2-nitrooxyethyl)-4-(2-N-methylaminoethoxy)-phthalimide

179. N-(2-nitrooxyethyl)-4-(2-N-ethylaminoethoxy)-phthalimide

180. N-(2-nitrooxypropyl)-4-[2-(1-pyrrolidinyl)-ethoxy]-5-nitrophthalimide

181. N-(2-nitrooxyethyl)-3-(3-nitrooxy-2-hydroxy-propoxy)phthalimide

182. N-(2-nitrooxyethyl)-3-(2,3-dinitrooxypropoxy)-phthalimide

183. N-(3-nitrooxypropyl)-3-(3-nitrooxy-2-hydroxy-propoxy)phthalimide

184. N-(3-nitrooxypropyl)-3-(2,3-dinitrooxypropoxy)-phthalimide

185. N-(2-nitrooxyethyl)-4-(2,3-dinitrooxypropoxy)-phthalimide

186. N-(2-nitrooxyethyl)-4-(2,3-dinitrooxypropoxy)-5-nitrophthalimide

187. N-(4-nitrooxybutyl)-4-[2-(2,6-dimethylpiperidino)-ethoxy]phthalimide

188. N-(5-nitrooxypentyl)-4-[2-(2,6-dimethyl-

piperidino)ethoxy]phthalimide

189. N-(2-nitrooxyethyl)-4-chlorophthalimide

190. N-(2-nitrooxyethyl)-4-(3-N,N-dimethylamino-propoxy)phthalimide nitrate

191. N-(2-nitrooxyethyl)-4-(3-N,N-diethylaminopropoxy)-phthalimide nitrate

192. N-(2-nitrooxyethyl)-3-(2-hydroxy-3-propylamino-propoxy)phthalimide

193. N-(2-nitrooxyethyl)-3-(2-hydroxy-3-isopropylamino-propoxy)phthalimide

194. N-(2-nitrooxyethyl)-3-(2-hydroxy-3-t-butylamino-propoxy)phthalimide

195. N-(3-nitrooxypropyl)-3-(2-hydroxy-3-t-butylamino-propoxy)phthalimide

196. N-(2-nitrooxyethyl)-4-(2-hydroxy-3-propylamino-propoxy)phthalimide

197. N-(2-nitrooxyethyl)-4-(2-hydroxy-3-isopropylamino-propoxy)phthalimide

198. N-(2-nitrooxyethyl)-4-(2-hydroxy-3-t-butylaminopropoxy)phthalimide

199. N-(2-nitrooxyethyl)-4-[2-hydroxy-3-(4-methyl-1-piperazinyl)propoxy]phthalimide

200. N-(2-nitrooxyethyl)-4-[2-hydroxy-3-(4-phenyl-1-piperazinyl)propoxy]phthalimide

201. N-(2-nitrooxyethyl)-4-[2-hydroxy-3-(4-p-tolyl-piperazinyl)propoxy]phthalimide

202. N-(2-nitrooxyethyl)-4-[2-hydroxy-3-(4-p-methoxyphenyl-1-piperazinyl)propoxy]phthalimide

203. N-(2-nitrooxyethyl)-4-[2-hydroxy-3-(4-p-chlorophenyl-1-piperazinyl)propoxy]phthalimide

204. N-(2-nitrooxyethyl)-4-[2-hydroxy-3-(4-ethoxycarbonyl-1-piperazinyl)propoxy]phthalimide

205. N-(2-nitrooxyethyl)-4-(2-hydroxy-3-morpholinopropoxy)phthalimide

206. N-(2-nitrooxyethyl)-4-(2-hydroxy-3-piperidinopropoxy)phthalimide

207. N-(2-nitrooxyethyl)-4-[2-hydroxy-3-(1-pyrrolidinyl)propoxy]phthalimide

208. N-(2-nitrooxyethyl)-4-(2-hydroxy-3-cyclohexylaminopropoxy)phthalimide

209. N-(2-nitrooxyethyl)-4-(2-hydroxy-3-benzylaminopropoxy)phthalimide

210. N-(2-nitrooxyethyl)-4-(2-nitrooxy-3-isopropylaminopropoxy)-5-nitrophthalimide

211. N-(2-nitrooxyethyl)-4-(2-hydroxy-3-t-butylaminopropoxy)-5-nitrophthalimide

212. N-(3-nitrooxypropyl)-4-(2-hydroxy-3-propylaminopropoxy)phthalimide

213. N-(3-nitrooxypropyl)-4-(2-hydroxy-3-isopropylaminopropoxy)phthalimide

214. N-(3-nitrooxypropyl)-4-(2-hydroxy-3-t-butylaminopropoxy)phthalimide

215. N-(2,3-dinitrooxypropyl)-4-(2-hydroxy-3-propylaminopropoxy)phthalimide

216. N-(2,3-dinitrooxypropyl)-4-(2-hydroxy-3-isopropylaminopropoxy)phthalimide

217. N-(2,3-dinitrooxypropyl)-4-(2-hydroxy-3-t-butylaminopropoxy)phthalimide

218. N-(2-hydroxy-3-nitrooxypropyl)-4-(2-N,N-dimethylaminoethoxy)phthalimide

219. N-(2-hydroxy-3-nitrooxypropyl)-4-(3-N,N-dimethylaminopropoxy)phthalimide

220. N-(2-hydroxy-3-nitrooxypropyl)-4-(3-N,N-diethylaminopropoxy)phthalimide

Of the compounds listed above, the most preferred compounds are Compounds No. 24, 51 and 52.

Compounds of formula (I) and salts thereof can be prepared by nitrating a compound of formula (II):

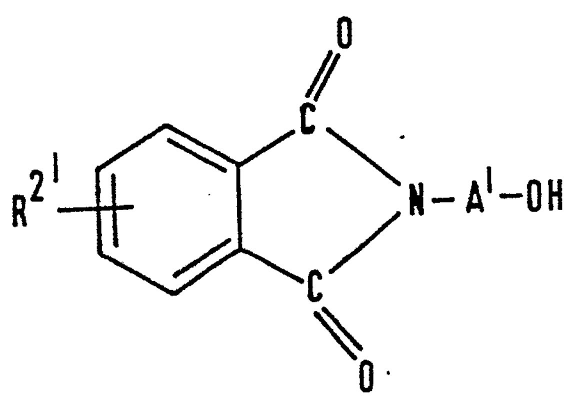

(II)

[in which A' represents a $C_1$-$C_6$ alkylene group or a $C_1$-$C_6$ alkylene group having 1 or 2 hydroxy substituents (i.e. the same as A, except that it may not have a nitrooxy substituent);

$R^{2'}$ represents a hydrogen atom, a $C_1$-$C_6$ alkoxy group, a $C_1$-$C_6$ alkoxy group having 1 or 2 hydroxy substituents, a hydroxy group, a $C_1$-$C_6$ alkanoylamino group, a halogen atom, a group of formula $R^3R^4N$-Y-B-O-, a group of formula $R^3R^4N-CH_2-CH(OH)-CH_2-O-$ or a 2,3-epoxypropoxy group (i.e. any of the groups or atoms which may be represented by $R^2$, except that the alkoxy group may not have a nitrooxy substituent and $R^5$ may not be a nitrooxy group, and that $R^{2'}$ can be a 2,3-epoxypropoxy group);

$R^3$, $R^4$, Y and B are as defined above]

and, if necessary, salifying the product.

In this reaction, the hydroxy group attached to the group represented by A' is nitrated, i.e. converted to a nitric acid ester (or nitrooxy) group. At the same time, depending upon the reaction conditions, one or more other hydroxy groups in the molecule of the compound of formula (II) may be converted to nitrooxy

groups and, where $R^{2'}$ represents a 2,3-epoxypropoxy group, this is converted (depending upon the reaction conditions) either to a 2-hydroxy-3-nitrooxypropoxy group or to a 2,3-di- nitrooxypropoxy group.

The reagent employed for nitration may be any conventional nitrating agent, for example: fuming nitric acid; a mixture of fuming nitric acid with acetic anhydride; or a mixture of concentrated nitric acid with concentrated sulphuric acid. The reaction may be effected in the presence or absence of a solvent. Where a solvent is employed, its nature is not critical to the invention, provided that it has no adverse effect upon the reaction. Suitable solvents which can be employed for this reaction include: ethers, such as diethyl ether, tetrahydrofuran or dioxane; esters of fatty acids, such as methyl acetate or ethyl acetate; ketones, such as acetone or methyl ethyl ketone; halogenated hydrocarbons, preferably halogenated aliphatic hydrocarbons, such as methylene chloride or chloroform; and nitriles, such as acetonitrile.

The reaction is preferably effected at a relatively low temperature, for example from -30°C to about ambient temperature. The time required for the reaction will vary, depending upon many factors, including the nature of the reagents and, most notably, the reaction

temperature, but a period of from 5 minutes to 10 hours will normally suffice.

Depending upon the reaction conditions, nitration of the benzene ring may also take place, to produce a compound of formula (I) in which $R^1$ represents a nitro group.

Alternatively, the nitration may be effected by first converting the compound of formula (II) to its corresponding halide (i.e. replacing the hydroxy group or some or all of the hydroxy groups by a halogen atom or atoms) and then reacting the resulting halide with silver nitrate. The conversion to the halide may be effected by conventional means employing a variety of well-known halogenating agents, for example thionyl chloride, sulphuryl chloride or oxalyl chloride. The reaction of the halide with silver nitrate is preferably effected in a solvent such as acetonitrile and at a temperature of from ambient temperature to 100°C. The time required for this reaction will vary, depending upon many factors, notably the reaction temperature, but a period of from 1 to 10 hours will normally suffice.

After completion of the reaction, the reaction product can be recovered from the reaction mixture by conventional means, for example by concentration under

reduced pressure, filtration and extraction. It may then, if desired, be further purified by conventional means, such as the various chromatography techniques, particularly column chromatography, or recrystallization.

Depending upon the reaction and recovery conditions, the resulting compound may (if it is basic) be obtained in the form of its nitrate. In this case, if the free base is required, this may be obtained by conventional means, by reaction with an alkali. Alternatively, the compound in the form of the free base may be salified by reaction with the chosen acid.

The starting material of formula (II) employed in the above reaction may be prepared by a variety of methods, the chosen method depending upon the nature of the compound of formula (I) which it is desired to prepare, as illustrated in the following Methods A, B and C.

Method A

Compounds of formula (I) in which $R^2$ represents a hydrogen atom, an unsubstituted $C_1$-$C_6$ alkoxy group, a hydroxy group, a $C_1$-$C_6$ alkanoylamino group or a halogen atom, that is to say compounds of formula (Ia), may be prepared as illustrated in the following reaction scheme:

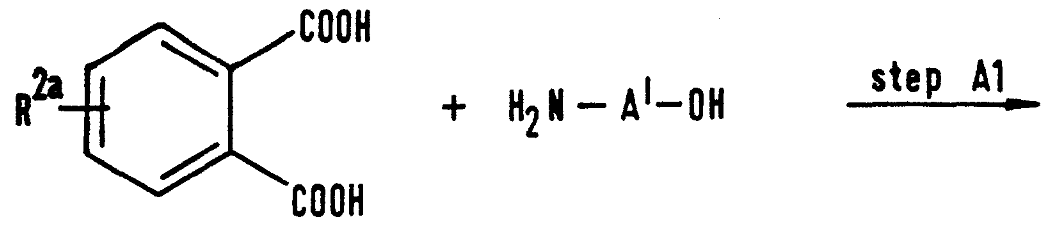


(III) (IV)

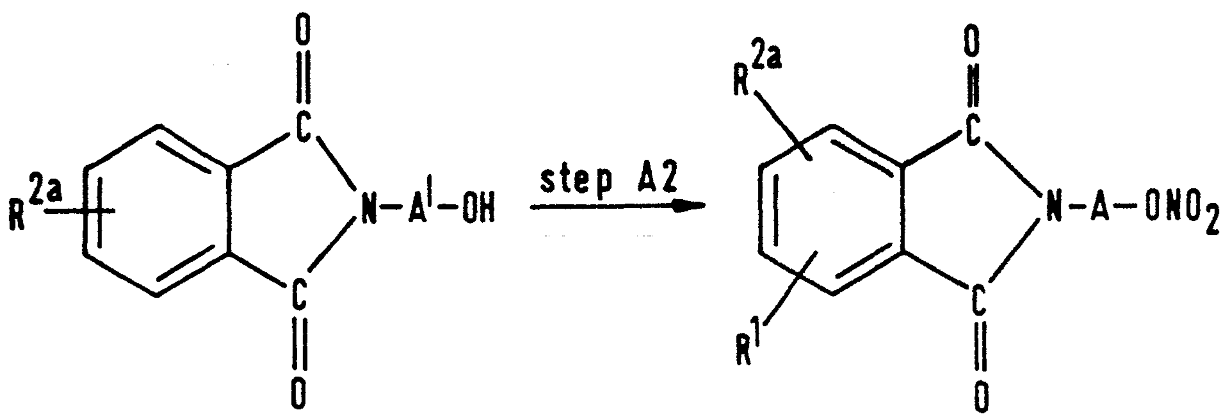


(V) (Ia)

In the above formulae, A, A' and $R^1$ are as defined above and $R^{2a}$ represents a hydrogen atom, a $C_1$-$C_6$ alkoxy group, a hydroxy group, a $C_1$-$C_6$ alkanoylamino group or a halogen atom.

Step A1 of this reaction comprises an imidation reaction of the phthalic acid or derivative thereof of formula (III) with a primary amine of formula (IV).

This reaction may be carried out under conditions well-known for preparing phthalimide derivatives, for example by dissolving the compounds of formulae (III) and (IV) in a suitable organic solvent and then distilling off the solvent and heating the residue. The nature of the solvent is not particularly critical, provided that it has no adverse effect on the reaction and that it can be distilled off at a temperature which does not harm the reagents or final product. Suitable solvents include alcohols, such as methanol or ethanol. The reaction temperature after distillation is preferably from 150 to 270°C. The time required for the reaction will vary, depending mainly upon the reaction temperature, but a period of from 10 minutes to 5 hours will usually suffice.

Step A2 of the above reaction scheme comprises the nitration reaction already described.

Method B

Compounds of formula (I) in which $R^2$ represents a group of formula $R^3R^4N-Y-B-O-$ or a $C_1-C_6$ alkoxy group having one or two hydroxy and/or nitrooxy substituents, that is to say compounds of formula (Ib), can be prepared by the method illustrated in the following reaction scheme:

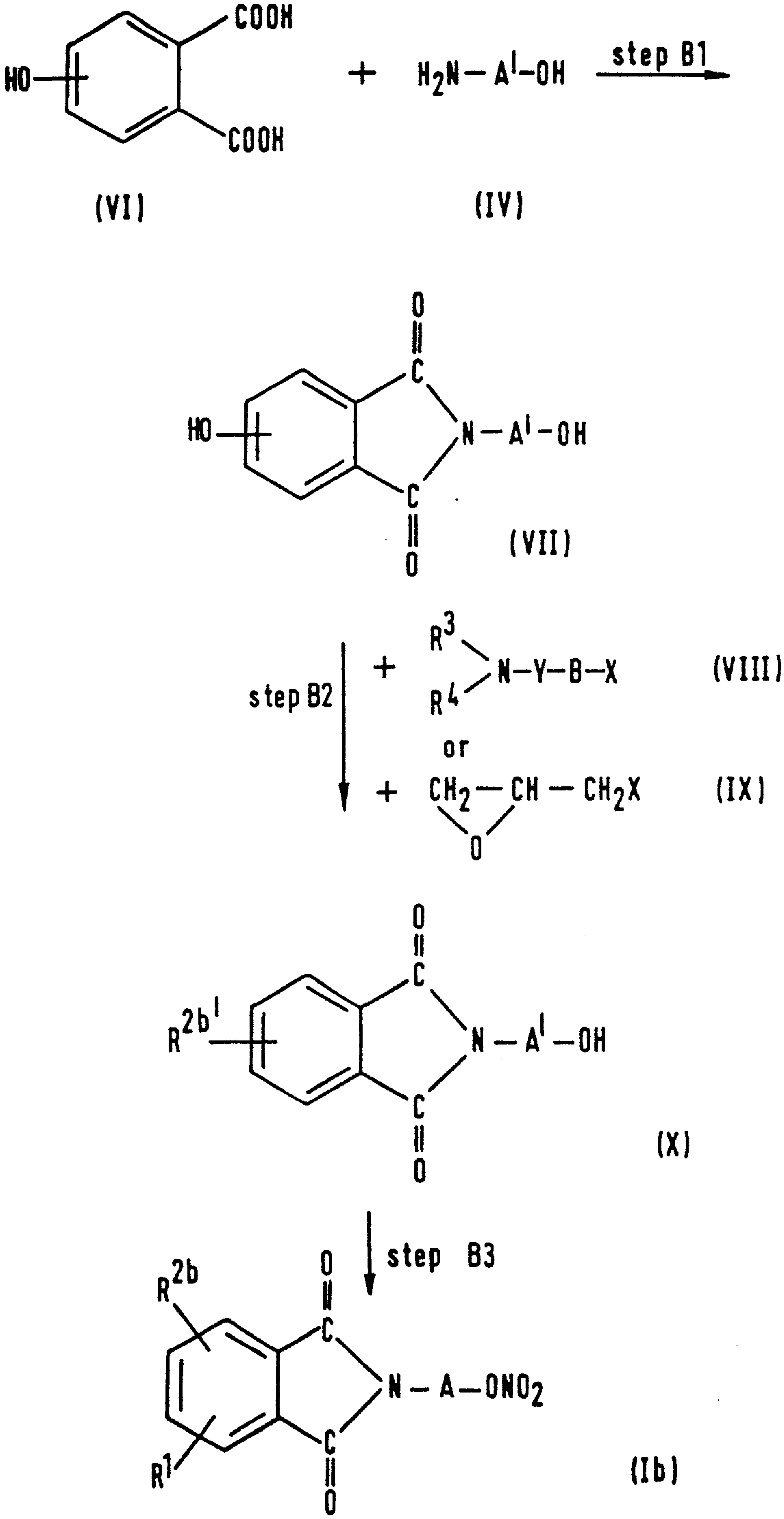


(VI) (IV) (VII) (VIII) (IX) (X) (Ib)

In the above formulae, A, A', $R^1$, $R^3$, $R^4$, Y and B are as defined above; $R^{2b}$ represents a group of formula $R^3R^4N$-Y-B-O- or a $C_1$-$C_6$ alkoxy group having one or two hydroxy and/or nitrooxy substituents; $R^{2b'}$ represents a group of formula $R^3R^4N$-Y-B-O- or a 2,3-epoxypropoxy group; and X represents a halogen atom, for example a chlorine, bromine or iodine atom.

The reaction of the hydroxyphthalic acid of formula (VI) with the primary amine (IV) in step B1 to prepare the compound of formula (VII) is the same imidation reaction and is carried out under the same conditions as the reaction in step A1 of Method A.

In step B2 of the reaction scheme, the resulting compound of formula (VII) is reacted with a substituted alkyl halide of formula (VIII) or (IX). This reaction is carried out under conditions well-known for the alkylation of aromatic hydroxy compounds, by reacting the compound of formula (VII) with the amino-substituted or carbamoyl-substituted alkyl halide (VIII) or the epihalohydrin (IX) in the presence of a base and preferably in the presence of a solvent. Where a solvent is employed, its nature is not critical, provided that it has no adverse effect upon the reaction. Suitable solvents include: ethers, such as tetrahydrofuran or dioxane; N,N-dimethylamides

of fatty acids, such as dimethylformamide or dimethylacetamide; ketones, such as acetone or methyl ethyl ketone; and alcohols, such as methanol or ethanol. A wide range of bases may be employed in this reaction, for example: alkali metal bicarbonates, such as sodium bicarbonate or potassium bicarbonate; alkali metal hydrides, such as sodium hydride; alkali metal alkoxides, such as sodium methoxide or sodium ethoxide; or organic bases, such as triethylamine or pyridine. The reaction will take place over a wide range of temperatures, for example from 0°C to 150°C, more preferably from ambient temperature to 110°C. The time required for the reaction will vary, depending upon many factors, primarily the reaction temperature, but a period of from 30 minutes to 20 hours will normally suffice.

In order to prepare compounds of formula (I) in which $R^2$ represents substituted alkoxy groups other than the 2,3-dihydroxypropoxy, 2,3-dinitrooxypropoxy and 2-hydroxy-3-nitrooxypropoxy groups, the reaction in step B2 may also be carried out employing a compound of formula $R^{b''}$-X (in which $R^{b''}$ represents a $C_2$-$C_6$ alkyl group having 1 or 2 hydroxy substituents and X is as defined above).

In step B3, the resulting compound of formula (X) is

nitrated, as previously described, to give the desired compound of formula (Ib).

Method C

Compounds of formula (I) in which $R^2$ represents a group of formula $R^3R^4N-CH_2-CH(R^5)-CH_2-O-$, that is to say compounds of formula (Ic), can be prepared by the reactions illustrated in the following reaction scheme:

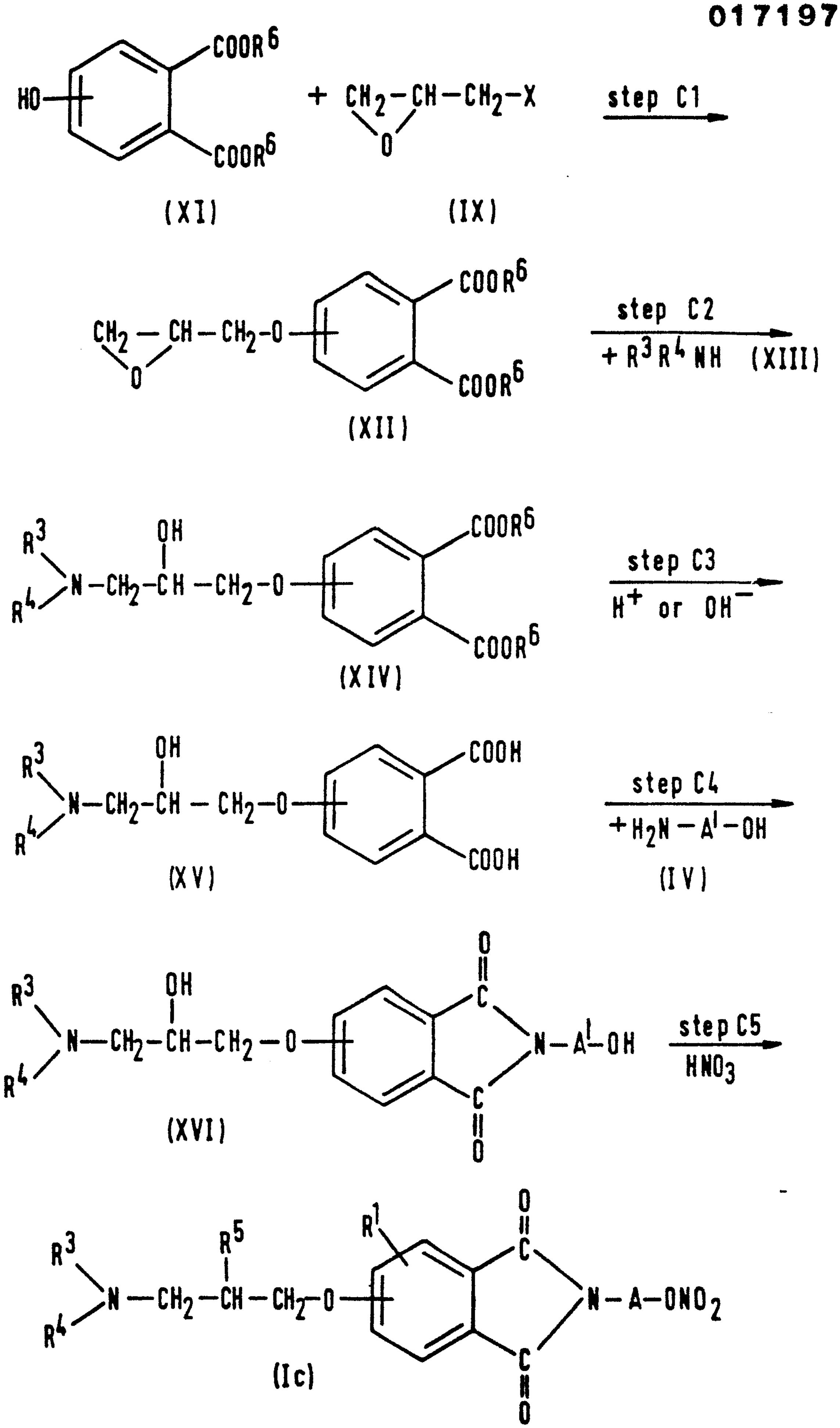


(XI) (IX)

(XII)

(XIV)

(XV)

(XVI)

(Ic)

In the above formulae, $R^1$, $R^3$, $R^4$, $R^5$, A, A' and X are as defined above and $R^6$ represents the alcoholic part of an ester, preferably a $C_1$-$C_6$ alkyl group, a $C_1$-$C_6$ alkyl group having from 1 to 3 substituents selected from the substituents defined above, an aralkyl group in which the aryl part is a $C_6$-$C_{10}$ carbocyclic aryl group (preferably a phenyl group) and the alkyl part is a $C_1$-$C_6$, preferably $C_1$-$C_3$ alkyl group (more preferably a methyl group), such an aralkyl group having from 1 to 3 of the substituents defined above, a diarylalkyl group in which each aryl part is a $C_6$-$C_{10}$ aryl (preferably phenyl) group and the alkyl part is a $C_1$-$C_6$, preferably $C_1$-$C_3$ alkyl (more preferably methyl) group, or such a diarylalkyl group having on each aryl part from 1 to 3 of the substituents defined above. Since, however, the groups represented by $R^6$ are eliminated in the course of the reaction scheme, their nature is not critical and any of these or any other alcoholic ester group may be chosen.

Step C1 of the reaction scheme is an identical reaction to the reaction of the compound of formula (VII) and the epihalohydrin (IX) in step B2 of Method B and takes place under the same conditions as does the reaction of step B2, to produce a compound of formula (XII).

This compound of formula (XII) is then reacted with the secondary amine of formula (XIII) in step C2, to give the compound of formula (XIV). This reaction is preferably effected in the presence of a solvent, the nature of which is not critical, provided that it has no adverse effect on the reaction. Suitable solvents include such alcohols as methanol or ethanol. The reaction will take place over a wide range of temperatures, but is preferably effected at ambient temperature or at the reflux temperature of the solvent employed, or at a temperature between these two. The time required for the reaction will vary, depending upon the reaction conditions, principally the reaction temperature, but a period of from 10 minutes to 10 hours will normally suffice.

In step C3, the ester of formula (XIV) obtained from step C2 is converted to the corresponding acid of formula (XV). This deesterification reaction may be carried out under conditions well-known for this type of reaction, for example by contacting the ester of formula (XIV) with an acid or a base in the presence of an aqueous solvent. The nature of the acid or base employed is not critical and any such acid or base commonly used in deesterification reactions may be used, for example: mineral acids, such as hydrochloric acid, hydrobromic acid, sulphuric acid or phosphoric acid; alkali metal hydroxides, such as potassium hydroxide or

sodium hydroxide; or alkali metal carbonates, such as sodium carbonate, or potassium carbonate. The nature of the aqueous solvent employed is not critical, provided that it has no adverse effect upon the reaction. Suitable solvents include: water itself; and mixtures of water with organic solvents, such as alcohols (for example methanol or ethanol). The reaction temperature is not critical and we normally find it convenient to carry out the reaction at about ambient temperature.

The reaction of the resulting acid of formula (XV) with the primary amine of formula (IV) is identical with the reaction in step A1 of Method A and may be carried out under identical conditions, to give the phthalimide derivative of formula (XVI). This is then subjected in step C5 to nitration, to give the desired compound of formula (Ic).

At any stage during the above reactions, the reaction product can be separated from the reaction mixture by a variety of conventional means (for example concentration by evaporation of the solvent under reduced pressure, filtration and extraction) and then, if desired, further purified by such conventional techniques as the chromatography techniques (e.g. column chromatography) or recrystallization. However, if desired, intermediate compounds may be employed for

subsequent reactions without purification and, in some cases, without intermediate isolation.

The biological activity of the compounds of the present invention is illustrated by the following tests.

Selective Vasodilative Effect on Collateral Blood Vessels

The test animals employed in this experiment were dogs. Each animal was anaesthetized with pentobarbital and the chest was opened to expose the heart, whilst respiration was maintained artificially.

Following the method of Winburg et al. [J. Pharmacol. Exp. Ther., 168, 70-95 (1969)], the vascular resistance ($R_L$) between two points, one at a large coronary artery on the cardiac surface and the other at a small coronary artery, was calculated by dividing the blood pressure difference between these two points by the blood flow. The peripheral resistance of the coronary artery ($R_T$) was determined and the value $R_L$ was divided by the value $R_T$, to give the selectivity ($R_L/R_T$).

Each compound of the invention tested was administered intravenously at a dose of 30 μg/kg body weight immediately prior to the measurement of vascular

resistance. The same experiment was carried out employing glyceryl trinitrate at a dose of 3 μg/kg body weight and the results are reported in the following Table 1 as the glyceryl trinitrate ratios, i.e. the ratio between the vascular resistance of the test compound and that of glyceryl trinitrate or the selectivity of the test compound and that of glyceryl trinitrate. In all cases, these ratios were found to be greater than 1.0, indicating that, at the doses given, the compounds of the invention had a greater selectivity for the large blood vessels than glyceryl trinitrate.

Table 1

| Compound No. | Glyceryl trinitrate ratio $R_L$ | $R_L/R_T$ |
|---|---|---|
| 25 | 1.6 | 1.6 |
| 27 | 1.8 | 1.4 |
| 51 | 1.6 | 1.4 |
| 54 | 1.7 | 2.1 |
| 59 | 1.2 | 1.6 |

Effect on Heart Beat

The right atrium of a guinea pig heart was excised and placed in a bath containing Krebs - Henseleit solution maintained at 37°C, through which was bubbled a gas containing 95% oxygen and 5% carbon dioxide. The spontaneous contraction of this right atrium was recorded, whilst the test compound was added, at concentrations between $10^{-5}$ and $10^{-4}$ g/ml, to the solution. In the case of glyceryl trinitrate, added at doses between $10^{-6}$ and $10^{-5}$ g/ml, very little or no change in the heart beat was observed. The results, in terms of the percentage decrease (-) or increase (+) in the rate of heart beat, for the compounds of the invention and for glyceryl trinitrate are shown in Table 2.

Table 2

| Compound No. | Concentration (g/ml) | | |
|---|---|---|---|
| | 1x10$^{-5}$ | 3x10$^{-5}$ | 1x10$^{-4}$ |
| 25 | -53% | -67% | -73% |
| 27 | -59% | -64% | -71% |
| 51 | -31% | -58% | -64% |
| 54 | -37% | -56% | -62% |
| 59 | -32% | -61% | -74% |
| Glyceryl trinitrate | 1x10$^{-6}$ | 3x10$^{-6}$ | 1x10$^{-5}$ |
| | 0% | +1.0% | 0% |

In intact animals, glyceryl trinitrate causes marked tarchycardia following intravenous administration. The results shown in Table 2, however, indicate that the compounds of the invention will not cause such a high degree of tachycardia because they have a direct inhibitory effect on the heart rate.

In the above tests, the compounds of the invention were employed at doses about 10 times the doses of glyceryl trinitrate employed for comparison. This indicates that the compounds of the invention are

somewhat less active than glyceryl trinitrate. However, it also demonstrates the fewer side effects of the compounds of the invention as compared with glyceryl trinitrate, since glyceryl trinitrate could not be used at, for example, such a high dose as 30 µg/kg because of severe side effects.

Moreover, the compounds of the invention show a greater selectivity for the large blood vessels ($R_L$ is a measure of the resistance of the large blood vessels). In the treatment of ischaemic heart diseases, the selective vasodilation of the collateral blood vessels (which are represented by the large blood vessels in the above tests) without affecting peripheral resistance is a most important factor. In a case where, for example, one of the coronary arteries is blocked, blood flow to the ischaemic area is supplied through the collateral blood vessels and the extent of this flow is determined by 2 factors. One of these factors is the resistance of the collateral blood vessels ($R_L$) and the other is the pressure head which drives blood to the ischaemic area. If a compound dilates peripheral blood vessels (whose resistance is represented by $R_T$), the pressure head (blood pressure) drops and blood flow to the ischaemic area correspondingly decreases. The results given above show that the compounds of the invention have a greater selectivity for large blood

vessels than for peripheral blood vessels, as compared with glyceryl trinitrate. Accordingly, they demonstrate that the compounds of the invention are likely to be more effective in the treatment of ischaemic heart disease than is glyceryl trinitrate.

Thus, the compounds of the invention have a selective vasodilatory effect upon the collateral blood vessels and exhibit little undesirable effect upon the heart beat. They are, therefore, useful in the treatment and prophylaxis of ischaemic heart diseases, notably angina pectoris.

For therapeutic administration, the compounds of the invention may be formulated with conventional carriers or diluents and in conventional pharmaceutical dosage forms, for example: for oral administration, as tablets, capsules, powders, fine granules, granules, solutions and suspensions; and for parenteral administration as injections and suppositories. The dosage employed will vary depending upon many factors, including the nature of the disorder, as well as the symptoms, age, condition and body weight of the patient and the route of administration. For oral administration to an adult human patient, a suitable daily dosage would be from 0.2 mg to 200 mg, more preferably from 15 mg to 60 mg, which may be administered in a single dose or in divided

doses. For non-oral administration, the preferred dose would be from 0.1 mg to 20 mg, more preferably from 2 mg to 6 mg, daily in a single dose.

The preparation of the compounds of the invention is illustrated by the following Examples. Preparation of certain of the starting materials employed in these Examples is illustrated by the following Preparations.

## PREPARATION 1

### N-(2-Hydroxyethyl)-4-hydroxyphthalimide

1.82 g (0.01 mole) of 4-hydroxyphthalic acid and 0.61 g (0.01 mole) of 2-aminoethanol were added to 20 ml of ethanol, and the solution was concentrated by evaporation under reduced pressure. The residue was heated at a temperature between 185 and 190°C for 50 minutes. At the end of this time, the reaction product was allowed to cool, giving 1.90 g (yield 92%) of the title compound as white needles. These were recrystallized from ethyl acetate to give the pure product melting at 161-162°C.

Elemental Analysis:

Calculated for $C_{10}H_9NO_4$:

C, 57.97%; H, 4.38%; N, 6.76%.

Found: C, 57.85%; H, 4.35%; N, 6.65%.

The following compounds were prepared by the same procedures as in Preparation 1.

N-(2-Hydroxyethyl)-3-hydroxyphthalimide

Melting at 171-173°C.

Elemental Analysis:

Calculated for $C_{10}H_9NO_4$:

C, 57.97%; H, 4.38%; N, 6.76%.

Found: C, 57.80%; H, 4.27%; N, 6.67%.

N-(3-Hydroxypropyl)-3-hydroxyphthalimide

Melting at 143-146°C.

Elemental Analysis:

Calculated for $C_{11}H_{11}NO_4$:

C, 59.72%; H, 5.01%; N, 6.33%.

Found: C, 59.50%; H, 4.96%; N, 6.44%.

N-(3-Hydroxypropyl)-4-hydroxyphthalimide

Melting at 129-131°C.

Elemental Analysis:

Calculated for $C_{11}H_{11}NO_4$:

C, 59.72%; H, 5.01%, N, 6.33%.

Found: C, 59.95%; H, 5.08%; N, 6.43%.

N-(4-Hydroxybutyl)-4-hydroxyphthalimide

Melting at 146-147°C.

Elemental Analysis:

Calculated for $C_{12}H_{13}NO_4$:

C, 61.27%; H, 5.57%; N, 5.96%.

Found: C, 61.00%; H, 5.58%; N, 5.92%.

N-(5-Hydroxypentyl)-4-hydroxyphthalimide

Melting at 118-120°C.

Elemental Analysis:

Calculated for $C_{13}H_{15}NO_4$:

C, 62.64%; H, 6.07%; N, 5.62%.

Found: C, 62.55%; H, 6.08%; N, 5.67%.

N-(2-Hydroxy-1-methylethyl)-4-hydroxyphthalimide

Melting at 148-152°C.

Elemental Analysis:

Calculated for $C_{11}H_{11}NO_4$:

C, 59.73%; H, 5.01%; N, 6.33%.

Found: C, 59.75%; H, 5.20%; N, 6.40%.

N-(2-Hydroxypropyl)-4-hydroxyphthalimide

Melting at 153-154°C.

Elemental Analysis:

Calculated for $C_{11}H_{11}NO_4$:

C, 59.72%; H, 5.01%; N, 6.33%.

Found: C, 59.63%; H, 5.15%; N, 6.42%.

PREPARATION 2

N-(2-Hydroxyethyl)-4-(2-N,N-diethylaminoethoxy)-phthalimide

At room temperature, 0.65 g (0.015 mole) of a 55% w/w suspension of sodium hydride in mineral oil was poured into 30 ml of anhydrous dimethylformamide, and the mixture was stirred for 30 minutes. 3.1 g (0.015 mole) of N-(2-hydroxyethyl)-4-hydroxyphthalimide (prepared as described in Preparation 1) were added slowly and the reaction mixture was stirred for a further 1 hour. 2.45 g (0.018 mole) of diethylaminoethyl chloride were added, after which the mixture was stirred for 1 hour at room temperature. The reaction mixture was then heated to 100°C and stirred for 5 hours. At the end of this time, the dimethylformamide was distilled off under reduced pressure. To the residue were added 50 ml of methylene chloride, and the mixture was stirred well. It was then filtered, and, after filtration, the filtrate was condensed by evaporation under reduced pressure, and the residue was purified by silica gel column chromatography, using a 10:9:1 by volume mixture of hexane, ethyl acetate and triethylamine as eluent, to afford 3.30 g (yield 71.7%) of the title compound as pale yellow crystals melting at 64-65°C.

Elemental Analysis:

Calculated for $C_{16}H_{22}N_2O_4$:
C, 62.72%; H, 7.24%; N, 9.14%.

Found: C, 62.54%; H, 7.19%; N, 9.19%.

## PREPARATION 3

### N-(2-Hydroxyethyl)-4-(2-morpholinoethoxy)phthalimide

A mixed solution of 4.1 g (0.02 mole) of *N*-(2-hydroxyethyl)-4-hydroxyphthalimide (prepared as described in Preparation 1), 2.8 g (0.02 mole) of

potassium carbonate, 3.6 g (0.024 mole) of N-(2-chloroethyl)morpholine and 100 ml of acetone was heated under reflux for 5 hours. The reaction mixture was cooled and then filtered, and the precipitate was washed with acetone. The washings and the filtrate were combined and condensed by evaporation under reduced pressure, and the residue was purified by silica gel column chromatography, using an 18:1:1 by volume mixture of ethyl acetate, ethanol and triethylamine as eluent. The oily substance obtained was crystallized from a mixture of ethyl acetate and hexane. 1.94 g (yield 30.6%) of the title compound was obtained as pale yellow plates melting at 106-107°C.

Elemental Analysis:

| | |
|---|---|
| Calculated for $C_{16}H_{20}N_2O_5$: | |
| | C, 59.99%; H, 6.29%; N, 8.75%. |
| Found: | C, 59.91%; H, 6.50%; N, 8.71%. |

## PREPARATION 4

### N-(2-Hydroxyethyl)-3-(2,3-epoxypropoxy)phthalimide

1.1 g of potassium carbonate, 2.1 g (0.015 mole) of epibromohydrin and 3.1 g (0.015 mole) of N-(2-hydroxyethyl)-3-hydroxyphthalimide (prepared following the procedure described in Preparation 1) were added to 40

ml of anhydrous dimethylformamide, and the mixture was stirred for 2 hours at a temperature between 60 and 70°C. The dimethylformamide was then distilled off under reduced pressure. The resulting residue was purified by silica gel column chromatography, using ethyl acetate as eluent, to give 1.35 g (yield 34.3%) of the title compound as white needles melting at 150-151°C.

Elemental Analysis:

Calculated for $C_{13}H_{13}NO_5$:

C, 59.31%; H, 4.98%; N, 5.32%.

Found: C, 59.32%; H, 4.87%; N, 5.10%.

The following compounds were also prepared by the processes described in Preparations 2, 3 and 4.

N-(2-Hydroxyethyl)-3-(2-diethylaminoethoxy)phthalimide

Melting at 103-105°C.

Elemental Analysis:

Calculated for $C_{16}H_{22}N_2O_4$:

C, 62.72%; H, 7.24%; N, 9.14%.

Found: C, 62.70%; H, 7.16%; N, 9.15%.

N-(3-Hydroxypropyl)-3-(2-diethylaminoethoxy)phthalimide

Melting at 58-61°C.

Elemental Analysis:

Calculated for $C_{17}H_{24}N_2O_4$:

C, 63.73%; H, 7.55%; N, 8.74%.

Found: C, 63.53%; H, 7.67%; N, 8.86%.

N-(3-Hydroxypropyl)-3-(2,3-epoxypropoxy)]phthalimide

Melting at 91-94°C.

Elemental Analysis:

Calculated for $C_{14}H_{15}NO_5$:

C, 60.64%; H, 5.45%; N, 5.05%.

Found: C, 60.69%; H, 5.37%; N, 4.90%.

N-(2-Hydroxyethyl)-4-(2-dimethylaminoethoxy)phthalimide

Melting at 94-95°C.

Elemental Analysis:

Calculated for $C_{14}H_{18}N_2O_4$:

C, 60.42%; H, 6.52%; N, 10.07%.

Found: C, 60.21%; H, 6.73%; N, 9.92%.

N-(2-Hydroxyethyl)-4-(3-dimethylaminopropoxy)phthalimide

Melting at 85-87°C.

Elemental Analysis:

Calculated for $C_{15}H_{20}N_2O_4$:

C, 61.63%; H, 6.90%; N, 9.58%.

Found: C, 61.61%; H, 6.71%; N, 9.50%.

N-(2-Hydroxyethyl)-4-(3-diethylaminopropoxy)phthalimide

An oil.

Elemental Analysis:

Calculated for $C_{17}H_{24}N_2O_4$:

C, 63.73%; H, 7.55%; N, 8.74%.

Found: C, 62.03%; H, 7.35%; N, 9.11%.

N-(2-Hydroxyethyl)-4-[(2S or 2R)-2-dimethylamino-propoxy)phthalimide

An oil.

Elemental Analysis:

Calculated for $C_{15}H_{20}N_2O_4$:

C, 61.63%; H, 6.90%; N, 9.58%.

Found: C, 61.67%; H, 7.03%; N, 9.28%.

N-(2-Hydroxyethyl)-4-[(2R or 2S)-2-dimethylamino-

propoxy)phthalimide

An oil.

Elemental Analysis:

Calculated for $C_{15}H_{20}N_2O_4$:

C, 61.63%; H, 6.90%; N, 9.58%.

Found: C, 61.34%; H, 7.12%; N, 9.60%.

N-(2-Hydroxyethyl)-4-(2-methyl-3-dimethylaminopropoxy)-phthalimide

An oil.

Elemental Analysis:

Calculated for $C_{16}H_{22}N_2O_4$:

C, 62.72%; H, 7.24%; N, 9.14%.

Found: C, 63.04%; H, 6.41%; N, 9.12%.

N-(2-Hydroxyethyl)-4-(2-diisopropylaminoethoxy)-phthalimide

Melting at 68-70°C.

Elemental Analysis:

Calculated for $C_{18}H_{26}N_2O_4$:

C, 64.65%; H, 7.84%; N, 8.38%.

Found: C, 65.04%; H, 7.63%; N, 8.61%.

N-(2-Hydroxyethyl)-4-[2-(N-benzyl-N-methylamino)ethoxy]-phthalimide

Melting at 79-81°C.

Elemental Analysis:

Calculated for $C_{20}H_{22}N_2O_4$:

C, 67.78%; H, 6.26%; N, 7.91%.

Found: C, 67.48%; H, 6.10%; N, 7.89%.

N-(2-Hydroxyethyl)-4-[2-(N-acetyl-N-methylamino)ethoxy]-phthalimide

Melting at 156-157°C.

Elemental Analysis:

Calculated for $C_{15}H_{18}N_2O_5$:

C, 58.81%; H, 5.92%; N, 9.15%.

Found: C, 58.52%; H, 5.91%; N, 9.22%.

N-(2-Hydroxyethyl)-4-[2-(1-pyrrolidinyl)ethoxy]-phthalimide

Melting at 116-119°C.

Elemental Analysis:

Calculated for $C_{16}H_{20}N_2O_4$:

C, 63.14%; H, 6.62%; N, 9.20%.

Found: C, 63.08%; H, 6.93%; N, 9.18%.

N-(3-Hydroxypropyl)-4-[2-(1-pyrrolidinyl)ethoxy]-phthalimide

Melting at 90-91°C.

Elemental Analysis:

Calculated for $C_{17}H_{22}N_2O_4$:

C, 64.13%; H, 6.97%; N, 8.80%.

Found: C, 64.11%; H, 6.87%; N, 8.77%.

N-(4-Hydroxybutyl)-4-[2-(1-pyrrolidinyl)ethoxy]-phthalimide

Melting at 89-90°C.

Elemental Analysis:

Calculated for $C_{18}H_{24}N_2O_4$:

C, 65.04%; H, 7.28%; N, 8.43%.

Found: C, 65.06%; H, 7.22%; N, 8.57%.

N-(5-Hydroxypentyl)-4-[2-(1-pyrrolidinyl)ethoxy]-phthalimide

An oil.

Elemental Analysis:

Calculated for $C_{19}H_{26}N_2O_4$:

C, 65.87%; H, 7.57%; N, 8.09%.

Found: C, 65.89%; H, 7.60%; N, 7.90%.

N-(2-Hydroxy-1-methylethyl)-4-[2-(1-pyrrolidinyl)ethoxy]-phthalimide

Melting at 89-91°C.

Elemental Analysis:

Calculated for $C_{17}H_{22}N_2O_4$:

C, 64.13%; H, 6.97%; N, 8.80%.

Found: C, 64.02%; H, 6.88%; N, 8.73%.

N-(2-Hydroxypropyl)-4-[2-(1-pyrrolidinyl)ethoxy]-phthalimide

Melting at 74-76°C.

Elemental Analysis:

Calculated for $C_{17}H_{22}N_2O_4$:

C, 64.13%; H, 6.97%; N, 8.80%.

Found: C, 63.95%; H, 6.89%; N, 8.79%.

N-(2-Hydroxyethyl)-4-[2-(2-methoxycarbonyl-1-pyrrolidinyl)ethoxy]phthalimide

An oil.

Elemental Analysis:

Calculated for $C_{18}H_{22}N_2O_6$:

C, 59.66%; H, 6.12%; N, 7.73%.

Found: C, 59.46%; H, 6.22%; N, 7.66%.

N-(2-Hydroxyethyl)-4-[(1-pyrrolidinyl)carbonylmethoxy]phthalimide

Melting at 145-146°C.

Elemental Analysis:

Calculated for $C_{16}H_{18}N_2O_5$:

C, 60.37%; H, 5.70%; N, 8.80%.

Found: C, 60.23%; H, 5.67%; N, 8.59%.

N-(2-Hydroxyethyl)-4-(2-piperidinoethoxy)phthalimide

Melting at 83-85°C.

Elemental Analysis:

Calculated for $C_{17}H_{22}N_2O_4$:

C, 64.13%; H, 6.79%; N, 8.80%.

Found: C, 63.88%; H, 6.89%; N, 8.65%.

N-(2-Hydroxyethyl)-4-(3-piperidinopropoxy)phthalimide

An oil.

Elemental Analysis:

Calculated for $C_{18}H_{24}N_2O_4$:

C, 65.04%; H, 7.28%; N, 8.43%.

Found: C, 64.87%; H, 7.44%; N, 8.50%.

N-(2-Hydroxyethyl)-4-[2-(4-ethoxycarbonyl-1-piperazinyl)-ethoxy]phthalimide

Melting at 105-107°C.

Elemental Analysis:

Calculated for $C_{19}H_{25}N_3O_6$:

C, 58.30%; H, 6.44%; N, 10.73%.

Found: C, 58.20%; H, 6.36%; N, 10.69%.

N-(2-Hydroxyethyl)-4-[2-(2,6-dimethylpiperidino)-ethoxy]phthalimide

Melting at 121-122°C.

Elemental Analysis:

Calculated for $C_{19}H_{26}N_{2}O_{4}$:

C, 65.87%; H, 7.57%; N, 8.09%.

Found: C, 66.37%; H, 7.87%; N, 7.91%.

## EXAMPLE 1

N-(2-Nitrooxyethyl)-4-(2-morpholinoethoxy)phthalimide (Compound No. 65)

To 10 ml of fuming nitric acid cooled to a temperature between -10 and -8°C were added in small portions 1.0 g (0.0031 mole) of N-(2-hydroxyethyl)-4-(2-morpholinoethoxy)phthalimide (prepared as described in Preparation 3). The reaction mixture was stirred for 30 minutes and then poured into ice-water. It was then neutralized with an aqueous solution of sodium bicarbonate and extracted with ethyl acetate. The extract was washed with water and dried. The dried extract was then condensed by evaporation under pressure, and the resulting residue was purified by silica gel column chromatography using a 19:1 by volume mixture of ethyl acetate and triethylamine as eluent. The product was crystallized from a mixture of ethyl acetate and hexane, to give 0.98 g (yield 86.0%) of the title compound as pale yellow plates melting at 104-105°C.

Elemental Analysis:

Calculated for $C_{16}H_{19}N_{3}O_{7}$:

C, 52.60%; H, 5.24%; N, 11.50%.

Found: C, 52.44%; H, 5.19%; N, 11.52%.

## EXAMPLE 2

N-(2-Nitrooxyethyl)-4-(2-N,N-diethylaminoethoxy)-phthalimide (Compound No. 25) and N-(2-nitrooxyethyl)-4-(2-N,N-diethylaminoethoxy)-5-nitrophthalimide (Compound No. 137)

0.8 g (0.0026 mole) of N-(2-hydroxyethyl)-4-(2-diethylaminoethoxy)phthalimide (prepared as described in Preparation 2) was added to 6 ml of fuming nitric acid cooled to a temperature between -10 and -8°C. The reaction mixture was stirred for 30 minutes, poured into ice-water, neutralized with an aqueous solution of sodium bicarbonate, and extracted with ethyl acetate. The extract was washed with water and dried, and then the solvent was removed by evaporation under reduced pressure. The residue was purified by silica gel column chromatography, using a 9:1 by volume mixture of methylene chloride and ethanol as eluent, to give 0.09 g (yield 8.7%) of Compound No. 137 (the 5-nitro compound) as a pale yellow powder melting at 107-108°C from the fractions first eluted.

Elemental Analysis:

Calculated for $C_{16}H_{20}N_4O_8$:

C, 48.48%; H, 5.09%; N, 14.14%.

Found: C, 48.47%; H, 5.01%; N, 14.11%.

Additionally, 0.48 g (yield 52.7%) of Compound No. 25 was obtained as a yellow powder melting at 50-52°C from fractions eluted subsequently.

Elemental Analysis:

Calculated for $C_{16}H_{21}N_3O_6$:

C, 54.69%; H, 6.02%; N, 11.96%.

Found: C, 54.49%; H, 5.87%; N, 11.94%.

## EXAMPLE 3

N-(2-Nitrooxyethyl)-3-(3-nitrooxy-2-hydroxypropoxy)-phthalimide (Compound No. 181)

1.3 g of N-(2-hydroxyethyl)-3-(2,3-epoxypropoxy)-phthalimide (prepared as described in Preparation 4) was added in small portions to 10 ml of fuming nitric acid cooled to a temperature between -10 and -8°C. The reaction mixture was stirred for 1 hour, and then poured into ice-water, neutralized with an aqueous solution of sodium bicarbonate and extracted with ethyl acetate. The extract was washed with water and dried, and then

the solvent was removed by evaporation under reduced pressure. The resulting residue was purified by silica gel column chromatography, using a 1:1 by volume mixture of ethyl acetate and benzene as eluent, to give 0.81 g of the title compound as a pale yellow oily substance.

Elemental Analysis:

Calculated for $C_{13}H_{13}N_3O_{10}$:

C, 42.05%; H, 3.53%; N, 11.32%.

Found: C, 42.34%; H, 3.79%; N, 11.41%.

By following the procedures described in Examples 1 to 3, the following compounds were also prepared. The compounds are identified herein by the Compound Nos. assigned to them in the foregoing list.

Compound No. 2

An oil.

Elemental Analysis:

Calculated for $C_{16}H_{21}N_3O_6$:

C, 54.69%; H, 6.02%; N, 11.96%.

Found: C, 54.47%; H, 5.94%; N, 11.66%.

Compound No. 14

Melting at 50-52°C.

Elemental Analysis:

Calculated for $C_{17}H_{23}N_3O_6$:

C, 55.88%; H, 6.35%; N, 11.50%.

Found: C, 55.67%; H, 6.23%; N, 11.86%.

Compound No. 183

An oil.

Elemental Analysis:

Calculated for $C_{14}H_{15}N_3O_{10}$:

C, 43.64%; H, 3.92%; N, 10.91%.

Found: C, 43.74%; H, 4.19%; N, 10.47%.

Compound No. 184

Melting at 79-82°C.

Elemental Analysis:

Calculated for $C_{14}H_{14}N_4O_{12}$:

C, 39.08%; H, 3.28%; N, 13.02%.

Found: C, 39.31%; H, 3.24%; N, 12.42%.

Compound No. 157

Melting at 86-87°C.

Elemental Analysis:

Calculated for $C_{10}H_8N_2O_5$:

C, 50.85%; H, 3.41%; N, 11.86%.

Found: C, 50.92%; H, 3.35%; N, 11.99%.

Compound No. 155

Melting at 250-251°C.

Elemental Analysis:

Calculated for $C_{10}H_7N_3O_7$:

C, 42.71%; H, 2.51%; N, 14.95%.

Found: C, 42.96%; H, 2.39%; N, 15.01%.

Compound No. 24

Melting at 71-72°C.

Elemental Analysis:

Calculated for $C_{14}H_{17}N_3O_6$:

C, 52.01%; H, 5.30%; N, 13.00%.

Found: C, 51.98%; H, 5.09%; N, 13.04%.

Compound No. 51

Melting at 60-63°C.

Elemental Analysis:

Calculated for $C_{15}H_{19}N_3O_6$:

C, 53.40%; H, 5.68%; N, 12.46%.

Found: C, 53.25%; H, 5.69%; N, 12.40%.

Compound No. 141

Melting at 80-83°C.

Elemental Analysis:

Calculated for $C_{15}H_{18}N_4O_8$:

C, 47.12%; H, 4.75%; N, 14.66%.

Found: C, 47.21%; H, 4.68%; N, 14.33%.

Compound No. 142

Melting at 68-70°C.

Elemental Analysis:

Calculated for $C_{15}H_{18}N_4O_8$:

C, 47.12%; H, 4.74%; N, 14.65%.

Found: C, 47.13%; H, 4.66%; N, 14.68%.

Compound No. 52

Melting at 44-45°C.

Elemental Analysis:

Calculated for $C_{17}H_{23}N_3O_6$:

C, 55.88%; H, 6.35%; N, 11.50%.

Found: C, 55.57%; H, 5.98%; N, 11.49%.

Compound No. 53

N-(2-nitrooxyethyl)-4-[(2R or 2S)-2-N,N-dimethylaminopropoxy]phthalimide

Melting at 77-78°C.

Elemental Analysis:

Calculated for $C_{15}H_{19}N_3O_6$:

C, 53.40%; H, 5.68%; N, 12.46%.

Found: C, 53.04%; H, 5.72%; N, 12.59%.

Compound No. 53

N-(2-nitrooxyethyl)-4-[(2S or 2R)-2-N,N-dimethylaminopropoxy]phthalimide

Melting at 58-60°C.

Elemental Analysis:

Calculated for $C_{15}H_{19}N_3O_6$:

C, 53.40%; H, 5.68%; N, 12.46%.

Found: C, 53.77%; H, 5.76%; N, 12.26%.

Compound No. 164

Melting at 155-157°C.

Elemental Analysis:

Calculated for $C_{10}H_8N_2O_6$:

C, 47.63%; H, 3.20%; N, 11.11%.

Found: C, 47.65%;; H, 3.17%; N, 11.02%.

Compound No. 54

An oil.

Elemental Analysis:

Calculated for $C_{16}H_{21}N_3O_6$:

C, 54.69%; H, 6.02%; N, 11.96%.

Found: C, 54.00%; H, 5.90%; N, 12.17%..

Compound No. 27

Melting at 87-88°C.

Elemental Analysis:

Calculated for $C_{18}H_{25}N_3O_6$:

C, 56.98%; H, 6.64%; N, 11.08%.

Found: C, 56.88%; H, 6.67%; N, 11.07%.

Compound No. 30

Melting at 72-74°C.

Elemental Analysis:

Calculated for $C_{20}H_{21}N_3O_6$:

C, 60.14%; H, 5.30%; N, 10.52%.

Found: C, 60.20%; H, 5.22%; N, 10.65%.

Compound No. 45

Melting at 123-125°C.

Elemental Analysis:

Calculated for $C_{15}H_{17}N_3O_7$:

C, 51.28%; H, 4.88%; N, 11.96%.

Found: C, 51.54%; H, 5.12%; N, 11.70%.

Compound No. 55

Melting at 95-97°C.

Elemental Analysis:

Calculated for $C_{16}H_{19}N_3O_6$:

C, 55.01%; H, 5.48%; N, 12.03%.

Found: C, 55.35%; H, 5.41%; N, 12.05%.

Compound No. 92

An oil.

Elemental Analysis:

Calculated for $C_{17}H_{21}N_3O_6$:

C, 56.19%; H, 5.83%; N, 11.56%.

Found: C, 56.40%; H, 5.48%; N, 11.24%.

Compound No. 150

Melting at 117-120°C.

Elemental Analysis:

Calculated for $C_{17}H_{20}N_4O_8$:

C, 50.00%; H, 4.94%; N, 13.72%.

Found: C, 49.92%; H, 4.81%; N, 13.36%.

Compound No. 116

Melting at 35-37°C.

Elemental Analysis:

Calculated for $C_{18}H_{23}N_3O_6$:

C, 57.28%; H, 6.14%; N, 11.14%.

Found: C, 57.04%; H, 5.99%; N, 11.18%.

Compound No. 122

Melting at 38-40°C.

Elemental Analysis:

Calculated for $C_{19}H_{25}N_3O_6$:

C, 58.30%; H, 6.44%; N, 10.74%.

Found: C, 58.63%; H, 6.62%; N, 10.99%.

Compound No. 127

Melting at 81-83°C.

Elemental Analysis:

Calculated for $C_{17}H_{21}N_3O_6$:

C, 56.19%; H, 5.83%; N, 11.57%.

Found: C, 56.26%; H, 5.73%; N, 11.84%.

Compound No 180

Melting at 139-141°C.

Elemental Analysis:

Calculated for $C_{17}H_{20}N_4O_8$:

C, 50.00%; H, 4.94%; N, 13.72%.

Found: C, 50.21%; H, 4.81%; N, 13.65%.

Compound No. 135

An oil.

Elemental Analysis:

Calculated for $C_{17}H_{20}N_4O_9$:

C, 48.11%; H, 4.75%; N, 13.20%.

Found: C, 48.14%; H, 4.87%; N, 13.04%.

Compound No. 130

Melting at 79-82°C.

Elemental Analysis:

Calculated for $C_{17}H_{21}N_3O_6$:

C, 56.19%; H, 5.83%; N, 11.57%.

Found: C, 56.11%; H, 5.77%; N, 11.55%.

Compound No. 58

An oil.

Elemental Analysis:

Calculated for $C_{17}H_{21}N_3O_6$:

C, 56.19%; H, 5.83%; N, 11.57%.

Found: C, 55.89%; H, 5.72%; N, 11.35%.

Compound No. 146

Melting at 82-84°C.

Elemental Analysis:

Calculated for $C_{17}H_{20}N_4O_8$:

C, 50.00%; H, 4.94%; N, 13.72%.

Found: C, 49.93%; H, 4.90%; N, 13.38%.

Compound No. 145

Melting at 91-93°C.

Elemental Analysis:

Calculated for $C_{17}H_{20}N_4O_8$:

C, 50.00%; H, 4.94%; N, 13.72%.

Found: C, 50.15%; H, 4.98%; N, 13.80%.

Compound No. 57

An oil.

Elemental Analysis:

Calculated for $C_{18}H_{21}N_3O_8$:

C, 53.07%; H, 5.20%; N, 10.32%.

Found: C, 52.89%; H, 5.05%; N, 10.05%.

Compound No. 49

Melting at 97-99°C.

Elemental Analysis:

Calculated for $C_{16}H_{17}N_3O_7$:

C, 52.89%; H, 4.72%; N, 11.57%.

Found: C, 52.82%; H, 4.71%; N, 11.70%.

Compound No. 59

Melting at 97-101°C.

Elemental Analysis:

Calculated for $C_{17}H_{21}N_3O_6$:

C, 56.19%; H, 5.83%; N, 11.56%.

Found: C, 56.13%; H, 5.63%; N, 11.50%.

Compound No. 64

An oil.

Elemental Analysis:

Calculated for $C_{18}H_{23}N_3O_6$:

C, 57.28%; H, 6.14%; N, 11.14%.

Found: C, 57.18%; H, 6.19%; N, 11.17%.

Compound No. 67

Melting at 59-62°C.

Elemental Analysis:

Calculated for $C_{19}H_{24}N_4O_8$:

C, 52.29%; H, 5.54%; N, 12.84%.

Found: C, 52.09%; H, 5.43%; N, 12.74%.

Compound No. 186

An oil.

Elemental Analysis:

Calculated for $C_{13}H_{11}N_5O_{14}.H_2O$:

C, 32.58%; H, 2.73%; N, 14.61%.

Found: C, 34.06%; H, 2.69%; N, 15.07%.

Compound No. 189

Melting at 109-110°C.

Compound No. 190

Melting at 90-92°C.

Compound No. 191

Melting at 100-101°C.

## EXAMPLE 4

N-(2-Nitrooxyethyl)-4-(2-hydroxy-3-propylaminopropoxy)-phthalimide (Compound No. 196)

(a) 10.2 g of potassium carbonate were added to a mixture of 10.5 g (0.05 mole) of dimethyl 4-hydroxyphthalate, 20.6 g (0.15 mole) of epibromohydrin and 300 ml of acetone, and the mixture was heated under reflux for 8 hours, with stirring. The reaction mixture was then allowed to stand overnight, and the potassium bromide formed was filtered off. The filtrate was concentrated by evaporation under reduced pressure, and then 150 ml of water were added to the residue, which was extracted with ethyl acetate. The extract was washed, in turn, with a 5% w/v aqueous solution of sodium hydroxide and with water, and then dried over anhydrous magnesium sulphate. The solvent was distilled off to leave an oil, which was then purified by column

chromatography through silica gel, eluted with a 1:1 by volume mixture of ethyl acetate and hexane, to give 6.1 g of dimethyl 4-(2,3-epoxypropoxy)phthalate in the form of an oil.

(b) A mixture of 2.7 g (0.01 mole) of dimethyl 4-(2,3-epoxypropoxy)phthalate [prepared as described in step (a) above], 0.6 g (0.01 mole) of propylamine and 20 ml of methanol was heated under reflux, with stirring, for 2 hours. The solvent was then removed by evaporation under reduced pressure, and 15 ml of water were added to the residue, which was then extracted with ethyl acetate. The extract was vigorously shaken with 30 ml of 1N hydrochloric acid, and the aqueous phase was separated, made alkaline by the addition of potassium carbonate, and then extracted with ethyl acetate. The extract was dried and the solvent was distilled off to leave an oil which was subjected to column chromatography through silica gel and eluted with ethanol to give crystals.

These crystals were recrystallized from a mixture of ethyl acetate and hexane to give 1.2 g of dimethyl 4-(2-hydroxy-3-propylaminopropoxy)phthalate as crystals melting at 58-59°C.

(c) A mixture of 0.98 g (3 mmole) of dimethyl

4-(2-hydroxy-3-propylaminopropoxy)phthalate [prepared as described in step (b) above], 19.8 ml of a 1N aqueous solution of sodium hydroxide and 20 ml of methanol was allowed to stand overnight at room temperature. 19.8 ml of 1N hydrochloric acid were then added to the reaction mixture and the solvent was distilled off. The residue was treated twice, each time with 50 ml of ethanol, and then water was removed by evaporation under reduced pressure. 30 ml of methanol were added to the residue, and the insolubles were filtered off. The methanol was distilled off under reduced pressure to give 1.0 g of 4-(2-hydroxy-3-propylaminopropoxy)phthalic acid in the form of a powder, which was then used for the next step without purification.

(d) 5.94 g (0.02 mole) of 4-(2-hydroxy-3-propylamino-propoxy)phthalic acid [prepared as described in step (c)] and 1.22 g (0.02 mole) of ethanolamine were dissolved in 250 ml of methanol, which was then distilled off under reduced pressure. The residue was heated on an oil bath kept at 200-205°C for 2 hours. At the end of this time, the reaction mixture was dissolved in 30 ml of water and then extracted five times, each time with 200 ml of ethyl acetate. The combined extracts were dried over anhydrous magnesium sulphate, and the ethyl acetate was distilled off under reduced pressure to give 1.9 g of <u>N</u>-(2-hydroxyethyl)-4-(2-hydroxy-3-propylaminopropoxy)phthalimide in the form of a powder melting at 104-107°C. This powder was then used in the next step without purification.

(e) 1 g of N-(2-hydroxyethyl)-4-(2-hydroxy-3-propylaminopropoxy)phthalimide [prepared as described in step (d)] was added, whilst stirring, to 10 ml of fuming nitric acid (specific gravity, 1.50) cooled at a temperature between -10 and -15°C, and the stirring was continued at that temperature for 40 minutes. At the end of this time, 50 g of ice-water were poured into the reaction mixture, which was then neutralized by the addition of sodium bicarbonate and extracted with ethyl acetate. The extract was washed with water and dried over anhydrous magnesium sulphate, and then the ethyl acetate was distilled off under reduced pressure. The residue was purified by column chromatography through silica gel, eluted with a 15:5:1 by volume mixture of ethyl acetate, ethanol and triethylamine, and then by recrystallization from ethyl acetate, to give 0.97 g of N-(2-nitrooxyethyl)-4-(2-hydroxy-3-propylaminopropoxy)phthalimide as crystals melting at 108-109°C.

Elemental Analysis:

Calculated for $C_{16}H_{21}N_3O_7$:
C, 52.31%; H, 5.76%; N, 11.44%.

Found: C, 52.44%; H, 5.87%; N, 11.32%.

0171977

CLAIMS:

1. Compounds of formula (I):

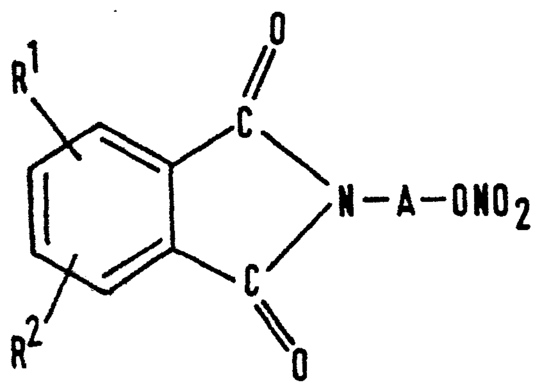

(I)

[wherein:

A represents a $C_1$-$C_6$ alkylene group, or a $C_1$-$C_6$ alkylene group having 1 or 2 hydroxy and/or nitrooxy substituents;

$R^1$ represents a hydrogen atom or the nitro group;

$R^2$ represents a hydrogen atom, a $C_1$-$C_6$ alkoxy group, a $C_1$-$C_6$ alkoxy group having 1 or 2 hydroxy and/or nitrooxy substituents, a hydroxy group, a $C_1$-$C_6$ alkanoylamino group, a halogen atom or a group of formula

$R^3R^4N-Y-B-O-$

or

$R^3R^4N-CH_2-CHR^5-CH_2-O-;$

$R^3$ represents a hydrogen atom or a $C_1-C_6$ alkyl group;

$R^4$ represents a hydrogen atom, a $C_1-C_6$ alkyl group, a $C_3-C_{10}$ cycloalkyl group, a $C_1-C_6$ alkanoyl group, a $C_6-C_{14}$ carbocyclic aryl group, a substituted $C_6-C_{14}$ carbocyclic aryl group, an aralkyl group in which the aryl part is $C_6-C_{14}$ carbocyclic aryl and the alkyl part is $C_1-C_6$ alkyl or a substituted aralkyl group in which the aryl part is $C_6-C_{14}$ carbocyclic aryl and the alkyl part is $C_1-C_6$ alkyl;

or $R^3$ and $R^4$, together with the nitrogen atom to which they are attached, represent a heterocyclic group having from 5 to 7 ring atoms of which 1 or 2, including the nitrogen atom to which $R^3$ and $R^4$ are attached, are nitrogen, oxygen or sulphur hetero-atoms, or such a heterocyclic group which is substituted;

$R^5$ represents a hydroxy or nitrooxy group;

B represents a $C_1$-$C_6$ alkylene group;

Y represents a carbonyl group or a direct bond between B and $-NR^3R^4$; and

the substituents on said substituted aryl, substituted aralkyl and substituted heterocyclic groups are from 1 to 3 substituents selected from $C_1$-$C_4$ alkyl groups, $C_1$-$C_4$ alkoxy groups, halogen atoms, nitro groups, carbamoyl groups, mono- and di-alkylcarbamoyl groups where the or each alkyl part is $C_1$-$C_4$ alkyl, carboxy groups, $C_2$-$C_7$ alkoxycarbonyl groups and, only for substituted heterocyclic groups, oxygen atoms, $C_6$-$C_{14}$ carbocyclic aryl groups, and substituted $C_6$-$C_{14}$ carbocyclic aryl groups];

and pharmaceutically acceptable salts thereof.

2. Compounds as claimed in Claim 1, wherein:

A represents an unsubstituted ethylene, propylene or trimethylene group;

$R^1$ represents a hydrogen atom or a nitro group;

$R^2$ represents, at the 3- or 4-position, a $C_2$-$C_4$

alkoxy group, a $C_2$-$C_4$ alkoxy group having 1 or 2 hydroxy and/or nitrooxy substituents, a hydroxy group, a $C_2$-$C_4$ alkanoylamino group, a halogen atom, a group of formula $R^3R^4N$-Y-B-O- or a group of formula $R^3R^4N-CH_2-CH(R^5)-CH_2-O-$;

$R^3$ represents a hydrogen atom or a $C_1$-$C_4$ alkyl group;

$R^4$ represents a hydrogen atom, a $C_1$-$C_4$ alkyl group, a $C_5$ or $C_6$ cycloalkyl group, a $C_2$-$C_4$ alkanoyl group, a phenyl group, a substituted phenyl group, or an aralkyl group in which the aryl part is a phenyl group or a substituted phenyl group and the alkyl part is a $C_1$-$C_3$ alkyl group;

or $R^3$ and $R^4$, together with the nitrogen atom to which they are attached, represent a heterocyclic group having 5 or 6 ring atoms of which 1 or 2, including the nitrogen atom to which $R^3$ and $R^4$ are attached, are nitrogen, oxygen or sulphur hetero-atoms, or such a heterocyclic group which is substituted;

$R^5$ represents a hydroxy or nitrooxy group;

B represents an ethylene, propylene or trimethylene

group;

Y represents a direct bond between the alkylene group represented by B and the group $-NR^3R^4$; and

the substituents are as defined in Claim 1;

and pharmaceutically acceptable salts thereof.

3. Compounds as claimed in Claim 1, wherein:

A represents an ethylene group;

$R^2$ represents said group of formula $R^3R^4N-Y-B-O-$ at the 3- or 4-position;

$R^3$ and $R^4$ both represent methyl groups or $R^3$ and $R^4$ both represent ethyl groups;

Y represents said direct bond; and

B represents an ethylene or trimethylene group;

and pharmaceutically acceptable salts thereof.

4. N-(2-Nitrooxyethyl)-4-(2-N,N-dimethylaminoethoxy)-phthalimide and pharmaceutically acceptable salts

thereof.

5. N-(2-Nitrooxyethyl)-4-(3-N,N-dimethylaminopropoxy)-phthalimide and pharmaceutically acceptable salts thereof.

6. N-(2-Nitrooxyethyl)-4-(3-N,N-diethylaminopropoxy)-phthalimide and pharmaceutically acceptable salts thereof.

7. A pharmaceutical composition comprising a vasodilator and a pharmaceutically acceptable carrier or diluent, wherein the vasodilator is at least one compound as claimed in any one of Claims 1 to 6.

8. A composition as claimed in Claim 7, formulated for oral administration.

9. A composition as claimed in Claim 7, formulated for parenteral administration.

10. The use of a compound as claimed in any one of Claims 1 to 6 for the manufacture of a medicament for the treatment or prophylaxis of ischaemic heart disease.

11. The use as claimed in Claim 10, wherein said medicament is for the treatment or prophylaxis of angina pectoris.

12. A process for preparing a compound as claimed in any one of Claims 1 to 6, which process comprises nitrating a compound of formula (II):

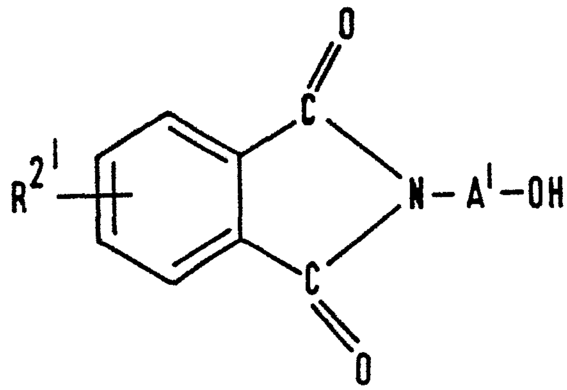

(II)

[in which A' represents a $C_1$-$C_6$ alkylene group or a $C_1$-$C_6$ alkylene group having 1 or 2 hydroxy substituents;

$R^{2'}$ represents a hydrogen atom, a $C_1$-$C_6$ alkoxy group, a $C_1$-$C_6$ alkoxy group having 1 or 2 hydroxy substituents, a hydroxy group, a $C_1$-$C_6$ alkanoylamino group, a halogen atom, a group of formula $R^3R^4N$-Y-B-O-, a group of formula $R^3R^4N$-$CH_2$-CH(OH)-$CH_2$-O- or a 2,3-epoxypropoxy group;

$R^3$, $R^4$, Y and B are as defined in Claim 1]

and, if necessary, salifying the product.

13. A process as claimed in Claim 12, wherein:

A' in said compound of formula (II) represents an unsubstituted ethylene, propylene or trimethylene group;

$R^{2'}$ in said compound of formula (II) represents, at the 3- or 4-position, a $C_2$-$C_4$ alkoxy group, a $C_2$-$C_4$ alkoxy group having 1 or 2 hydroxy substituents, a hydroxy group, a $C_2$-$C_4$ alkanoylamino group, a halogen atom, a group of formula $R^3R^4N$-Y-B-O-, a group of formula $R^3R^4N-CH_2-CH(OH)-CH_2-O-$ or a 2,3-epoxypropoxy group; and

$R^3$, $R^4$, B and Y are as defined in Claim 2; and

the nitration is effected either to the stage where only the hydroxy group attached to the group represented by A' is nitrated or to the stage where that hydroxy group and one or more hydroxy groups forming substituents on the group represented by $R^{2'}$ is nitrated, to prepare a compound as claimed in Claim 2.

14. A process as claimed in Claim 12, wherein <u>N</u>-(2-hydroxyethyl)-4-(2-<u>N</u>,<u>N</u>-dimethylaminoethoxy)-phthalimide is nitrated, to prepare <u>N</u>-(2-nitrooxyethyl)-4-(2-<u>N</u>,<u>N</u>-dimethylaminoethoxy)phthalimide or a

pharmaceutically acceptable salt thereof.

15. A process as claimed in Claim 12, wherein N-(2-hydroxyethyl)-4-(3-N,N-dimethylaminopropoxy)phthalimide is nitrated, to prepare N-(2-nitrooxyethyl)-4-(3-N,N-dimethylaminopropoxy)phthalimide or a pharmaceutically acceptable salt thereof.

16. A process as claimed in Claim 12, wherein N-(2-hydroxyethyl)-4-(3-N,N-diethylaminopropoxy)phthalimide is nitrated, to prepare N-(2-nitrooxyethyl)-4-(3-N,N-diethylaminopropoxy)phthalimide or a pharmaceutically acceptable salt thereof.

CLAIMS:

1. A process for preparing compounds of formula (I):

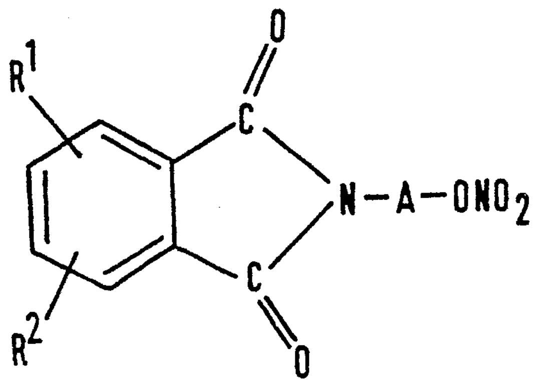

(I)

[wherein:

A represents a $C_1-C_6$ alkylene group, or a $C_1-C_6$ alkylene group having 1 or 2 hydroxy and/or nitrooxy substituents;

$R^1$ represents a hydrogen atom or a nitro group;

$R^2$ represents a hydrogen atom, a $C_1-C_6$ alkoxy group, a $C_1-C_6$ alkoxy group having 1 or 2 hydroxy and/or nitrooxy substituents, a hydroxy group, a $C_1-C_6$ alkanoylamino group, a halogen atom or a group

of formula

$$R^3R^4N\text{-}Y\text{-}B\text{-}O\text{-}$$

or

$$R^3R^4N\text{-}CH_2\text{-}CHR^5\text{-}CH_2\text{-}O\text{-};$$

$R^3$ represents a hydrogen atom or a $C_1$-$C_6$ alkyl group;

$R^4$ represents a hydrogen atom, a $C_1$-$C_6$ alkyl group, a $C_3$-$C_{10}$ cycloalkyl group, a $C_1$-$C_6$ alkanoyl group, a $C_6$-$C_{14}$ carbocyclic aryl group, a substituted $C_6$-$C_{14}$ carbocyclic aryl group, an aralkyl group in which the aryl part is $C_6$-$C_{14}$ carbocyclic aryl and the alkyl part is $C_1$-$C_6$ alkyl or a substituted aralkyl group in which the aryl part is $C_6$-$C_{14}$ carbocyclic aryl and the alkyl part is $C_1$-$C_6$ alkyl;

or $R^3$ and $R^4$, together with the nitrogen atom to which they are attached, represent a heterocyclic group having from 5 to 7 ring atoms of which 1 or 2, including the nitrogen atom to which $R^3$ and $R^4$ are attached, are nitrogen, oxygen or sulphur hetero-atoms, or such a heterocyclic group which is substituted;

$R^5$ represents a hydroxy or nitrooxy group;

B represents a $C_1$-$C_6$ alkylene group;

Y represents a carbonyl group or a direct bond between B and $-NR^3R^4$; and

the substituents on said substituted aryl, substituted aralkyl and substituted heterocyclic groups are from 1 to 3 substituents selected from $C_1$-$C_4$ alkyl groups, $C_1$-$C_4$ alkoxy groups, halogen atoms, nitro groups, carbamoyl groups, mono- and di-alkylcarbamoyl groups where the or each alkyl part is $C_1$-$C_4$ alkyl, carboxy groups, $C_2$-$C_7$ alkoxycarbonyl groups and, only for substituted heterocyclic groups, oxygen atoms, $C_6$-$C_{14}$ carbocyclic aryl groups, and substituted $C_6$-$C_{14}$ carbocyclic aryl groups];

and pharmaceutically acceptable salts thereof,

which process comprises nitrating a compound of formula (II):

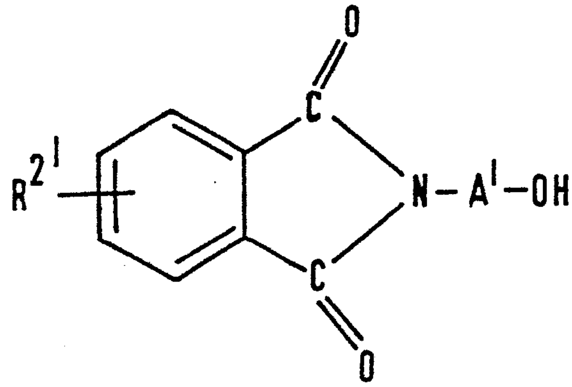

(II)

[in which A' represents a $C_1$-$C_6$ alkylene group or a $C_1$-$C_6$ alkylene group having 1 or 2 hydroxy substituents;

$R^{2'}$ represents a hydrogen atom, a $C_1$-$C_6$ alkoxy group, a $C_1$-$C_6$ alkoxy group having 1 or 2 hydroxy substituents, a hydroxy group, a $C_1$-$C_6$ alkanoylamino group, a halogen atom, a group of formula $R^3R^4N$-Y-B-O-, a group of formula $R^3R^4N-CH_2-CH(OH)-CH_2-O-$ or a 2,3-epoxypropoxy group;

$R^3$, $R^4$, Y and B are as defined above]

and, if necessary, salifying the product.

2. A process as claimed in Claim 1, wherein:

A' in said compound of formula (II) represents an unsubstituted ethylene, propylene or trimethylene group;

$R^{2'}$ in said compound of formula (II) represents, at the 3- or 4-position, a $C_2$-$C_4$ alkoxy group, a $C_2$-$C_4$ alkoxy group having 1 or 2 hydroxy substituents, a hydroxy group, a $C_2$-$C_4$ alkanoylamino group, a halogen atom, a group of formula $R^3R^4N$-Y-B-O-, a group of formula $R^3R^4N-CH_2-CH(OH)-CH_2-O-$ or a 2,3-epoxypropoxy group;

$R^3$ in said compound of formula (II) represents a hydrogen atom or a $C_1$-$C_4$ alkyl group;

$R^4$ in said compound of formula (II) represents a hydrogen atom, a $C_1$-$C_4$ alkyl group, a $C_5$ or $C_6$ cycloalkyl group, a $C_2$-$C_4$ alkanoyl group, a phenyl group, a substituted phenyl group, or an aralkyl group in which the aryl part is a phenyl group or a substituted phenyl group and the alkyl part is a $C_1$-$C_3$ alkyl group;

or $R^3$ and $R^4$, together with the nitrogen atom to which they are attached in said compound of formula

(II), represent a heterocyclic group having 5 or 6 ring atoms of which 1 or 2, including the nitrogen atom to which $R^3$ and $R^4$ are attached, are nitrogen, oxygen or sulphur hetero-atoms, or such a heterocyclic group which is substituted;

B in said compound of formula (II) represents an ethylene, propylene or trimethylene group;

Y in said compound of formula (II) represents a direct bond between the alkylene group represented by B and the group $-NR^3R^4$; and

the nitration is effected either to the stage where only the hydroxy group attached to the group represented by A' is nitrated or to the stage where that hydroxy group and one or more hydroxy groups forming substituents on the group represented by $R^{2'}$ is nitrated.

3. A process as claimed in Claim 1, wherein:

A' in said compound of formula (II) represents an ethylene group;

$R^{2'}$ in said compound of formula (II) represents said

group of formula $R^3R^4N-Y-B-O-$ at the 3- or 4-position;

$R^3$ and $R^4$ in said compound of formula (II) both represent methyl groups or $R^3$ and $R^4$ in said compound of formula (II) both represent ethyl groups;

Y in said compound of formula (II) represents said direct bond; and

B in said compound of formula (II) represents an ethylene or trimethylene group.

4. A process as claimed in Claim 1, wherein N-(2-hydroxyethyl)-4-(2-N,N-dimethylaminoethoxy)-phthalimide is nitrated, to prepare N-(2-nitrooxyethyl)-4-(2-N,N-dimethylaminoethoxy)phthalimide or a pharmaceutically acceptable salt thereof.

5. A process as claimed in Claim 1, wherein N-(2-hydroxyethyl)-4-(3-N,N-dimethylaminopropoxy)phthalimide is nitrated, to prepare N-(2-nitrooxyethyl)-4-(3-N,N-dimethylaminopropoxy)phthalimide or a pharmaceutically acceptable salt thereof.

6. A process as claimed in Claim 1, wherein N-(2-hydroxyethyl)-4-(3-N,N-diethylaminopropoxy)phthalimide

is nitrated, to prepare N-(2-nitrooxyethyl)-4-(3-N,N-diethylaminopropoxy)phthalimide or a pharmaceutically acceptable salt thereof.

7. The use of a compound of formula (I), as defined in Claim 1, or a pharmaceutically acceptable salt thereof for the manufacture of a medicament for the treatment or prophylaxis of ischaemic heart disease.

8. The use as claimed in Claim 7, wherein there is used a compound in which:

A represents an unsubstituted ethylene, propylene or trimethylene group;

$R^1$ represents a hydrogen atom or a nitro group;

$R^2$ represents, at the 3- or 4-position, a $C_2$-$C_4$ alkoxy group, a $C_2$-$C_4$ alkoxy group having 1 or 2 hydroxy and/or nitrooxy substituents, a hydroxy group, a $C_2$-$C_4$ alkanoylamino group, a halogen atom, a group of formula $R^3R^4N$-Y-B-O- or a group of formula $R^3R^4N-CH_2-CH(R^5)-CH_2-O-$;

$R^3$ represents a hydrogen atom or a $C_1$-$C_4$ alkyl group;

$R^4$ represents a hydrogen atom, a $C_1$-$C_4$ alkyl group, a $C_5$ or $C_6$ cycloalkyl group, a $C_2$-$C_4$ alkanoyl group, a phenyl group, a substituted phenyl group, or an aralkyl group in which the aryl part is a phenyl group or a substituted phenyl group and the alkyl part is a $C_1$-$C_3$ alkyl group;

or $R^3$ and $R^4$, together with the nitrogen atom to which they are attached, represent a heterocyclic group having 5 or 6 ring atoms of which 1 or 2, including the nitrogen atom to which $R^3$ and $R^4$ are attached, are nitrogen, oxygen or sulphur hetero-atoms, or such a heterocyclic group which is substituted;

$R^5$ represents a hydroxy or nitrooxy group;

B represents an ethylene, propylene or trimethylene group; and

Y represents a direct bond between the alkylene group represented by B and the group $-NR^3R^4$;

or a pharmaceutically acceptable salt thereof.

9. The use as claimed in Claim 7, wherein there is used a compound in which:

A represents an ethylene group;

$R^2$ represents said group of formula $R^3R^4N-Y-B-O-$ at the 3- or 4-position;

$R^3$ and $R^4$ both represent methyl groups or $R^3$ and $R^4$ both represent ethyl groups;

Y represents said direct bond; and

B represents an ethylene or trimethylene group;

or a pharmaceutically acceptable salt thereof.

10. The use as claimed in Claim 7, wherein said compound is N-(2-nitrooxyethyl)-4-(2-N,N-dimethylamino-ethoxy)phthalimide or a pharmaceutically acceptable salt thereof.

11. The use as claimed in Claim 7, wherein said compound is N-(2-nitrooxyethyl)-4-(3-N,N-dimethylamino-propoxy)phthalimide or a pharmaceutically acceptable salt thereof.

12. The use as claimed in Claim 7, wherein said compound is N-(2-nitrooxyethyl)-4-(3-N,N-diethylamino-propoxy)phthalimide or a pharmaceutically acceptable

salt thereof.

13. The use as claimed in any one of Claims 7 to 12, wherein said medicament is formulated for oral administration.

14. The use as claimed in any one of Claims 7 to 12, wherein said medicament is formulated for parenteral administration.

15. The use as claimed in any one of Claims 7 to 14, wherein said medicament is for the treatment or prophylaxis of angina pectoris.

16. A process for preparing a compound of formula (I), as defined in Claim 1, or a pharmaceutically acceptable salt thereof, by methods known for analogous compounds.

European Patent Office

# EUROPEAN SEARCH REPORT

0171977
Application number

EP 85305471.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF POLYMER SCIENCE, vol. 21, no. 10, October 1983<br>OVERBERGER C.G. et al. "Syntheses and Polymerizations of Some Vinyl Monomers Containing Nitro- or Fluorophthalimides" pages 3403-3424<br>* Pages 3405,3414,3415 *<br>-- | 1,12 | C 07 D 209/48<br>C 07 D 401/12<br>C 07 D 403/12<br>C 07 D 413/12<br>C 07 D 417/12<br>A 61 K 31/395 |
| A | US - A - 3 984 435 (KAZUO MATSUI et al.)<br>* Abstract; column 13, compound no. 29; column 17, compound no. 49; column 19, compound no. 66 *<br>---- | 1 | |
| | | | **TECHNICAL FIELDS SEARCHED (Int. Cl.4)** |
| | | | C 07 D 209/00<br>C 07 D 401/00<br>C 07 D 403/00<br>C 07 D 413/00<br>C 07 D 417/00 |

The present search report has been drawn up for all claims

| Place of search | Date of completion of the search | Examiner |
|---|---|---|
| VIENNA | 10-10-1985 | HEIN |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82